(19) Europäisches Patentamt
European Patent Office
Office européen des brevets

(11) Veröffentlichungsnummer: **0 313 974**
**A1**

(12) # EUROPÄISCHE PATENTANMELDUNG

(21) Anmeldenummer: **88117369.4**

(22) Anmeldetag: **19.10.88**

(51) Int. Cl.4: **C07D 211/46 , C07D 401/12 , C07D 401/06 , C07D 405/12 , C07D 453/02 , C07D 409/12 , A61K 31/445 , A61K 31/505 , A61K 31/47**

(30) Priorität: **30.10.87 DE 3736771**
**29.04.88 DE 3814549**

(43) Veröffentlichungstag der Anmeldung:
**03.05.89 Patentblatt 89/18**

(84) Benannte Vertragsstaaten:
**AT BE CH DE ES FR GB GR IT LI LU NL SE**

(71) Anmelder: **BAYER AG**
**Konzernverwaltung RP Patentabteilung**
**D-5090 Leverkusen 1 Bayerwerk(DE)**

(72) Erfinder: **Böshagen, Horst, Dr.**
**Wiesenstrasse 4**
**D-5657 Haan(DE)**
Erfinder: **Junge, Bodo, Dr.**
**Spieckern 23**
**D-5600 Wuppertal 23(DE)**
Erfinder: **Paessens, Arnold, Dr.**
**Stresemannstrasse 56**
**D-5657 Haan(DE)**
Erfinder: **Schüller, Matthias, Dr.**
**Gellertweg 20**
**D-5600 Wuppertal 1(DE)**

(54) **N-substituierte Derivate von 1-Desoxynojirimycin und 1-Desoxymannonojirimycin, Verfahren zu deren Herstellung und deren Verwendung in Arzneimitteln.**

(57) Die Erfindung betrifft N-substituierte Derivate von 1-Desoxy-nojirimycin und 1-Desoxy-mannonojirimycin, Verfahren zu ihrer Herstellung und ihre Verwendung in Arzneimitteln, insbesondere in antiviralen Arzneimitteln.

EP 0 313 974 A1

# N-substituierte Derivate von 1-Desoxynojirimycin und 1-Desoxymannonojirimycin, Verfahren zu deren Herstellung und deren Verwendung in Arzneimitteln

Die Erfindung betrifft N-substituierte Derviate von 1-Desoxy-nojirimycin und 1-Desoxy-mannonojirimycin, Verfahren zu ihrer Herstellung und ihre Verwendung in Arzneimitteln, insbesondere in antiviralen Arzneimitteln.

Es ist bekannt, daß Derivate von 1-Desoxy-nojirimycin und 1-Desoxy-mannonojirimycin Glukosidase-Inhibitoren sind und zur Behandlung von Kohlenhydratstoffwechselerkrankungen eingesetzt werden [EP 947; EP 80 58; EP 34 784; EP 28 48 117; EP 193 770]. Darüberhinaus ist bekannt, daß bestimmte 1-Desoxynojirimycin-Derviate herbizide Eigenschaften aufweisen [DE 30 24 901] und 1-Desoxy-nojirimycin und 1-Desoxy-mannojirimycin und Derivate davon die Biosynthese von Glykoproteinen beeinflussen [U. Fuhrmann, E. Bauss, H. Ploegh; Review; Biochimica et Biophysica Acta 825, 95-110 (1985)].

Die vorliegende Erfindung betrifft N-substituierte Derivate von 1-Desoxy-nojirimycin und 1-Desoxy-mannojirimycin der allgemeinen Formel (I)

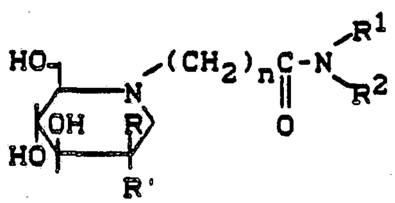

(I)

in welcher

R oder R′ für eine Hydroxylgruppe steht und der jeweils andere, R′ oder R, für Wasserstoff steht,

n - für eine Zahl 1 bis 6 steht,

$R^1$ - für Wasserstoff steht, oder

- für geradkettiges oder verzweigtes Alkyl mit bis zu 3 Kohlenstoffatomen oder für Benzyl steht,

und

$R^2$ - für geradkettiges oder verzweigtes Alkyl mit bis zu 8 Kohlenstoffatomen steht, das substituiert sein kann durch Aryl mit 6 bis 10 Kohlenstoffatomen, wobei der Arylrest bis zu 4 gleiche oder verschiedene Substituenten der Reihe Fluor, Chlor, Brom, Iod, Cyano, Nitro, Alkyl, Alkoxy, Alkythio oder Arylthio mit jeweils bis zu 6 Kohlenstoffatomen, Dioxymethylen, Dioxyethylen, Trifluormethyl, Trifluor methyl, Trifluormethoxy, Difluormethoxy, Carboxy oder Alkoxycarbonyl mit bis zu 4 Kohlenstoffatomen tragen kann, oder durch Pyridyl, Thienyl, Furyl, Pyrimidyl, Pyridazinyl, Pyrazinyl oder Chinolyl substituiert sein kann, oder

- für Cycloalkyl mit 3 bis 7 Kohlenstoffatomen steht, oder

- für Aryl mit 6 bis 10 Kohlenstoffatomen steht, wobei der Arylrest bis zu 4-fach gleich oder verschieden substituiert sein kann durch Fluor, Chlor, Brom, Iod, Cyano, Alkyl mit bis zu 6 Kohlenstoffatomen, Alkyloxy mit bis zu 12 Kohlenstoffatomen, Alkylsulfonyl mit jeweils bis zu 4 Kohlenstoffatomen, Trifluormethyl, Trifluormethoxy, Difluormethylen, Difluormethoxy, Trifluormethylthio, Dioxymethylen, Dioxyethylen, durch Cycloalkyl mit 3 bis 7 Kohlenstoffatomen, durch Carboxy oder Alkoxycarbonyl mit bis zu 4 Kohlenstoffatomen, durch Aryloxy mit 6 bis 10 Kohlenstoffatomen, wobei der Arylrest bis zu 4 gleiche oder verschiedene Substituenten der Reihe Fluor, Chlor, Brom, Iod, Cyano, Hydroxy, Nitro, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder durch Halogenalkoxy mit bis zu 6 Kohlenstoffatomen und bis zu 5 Fluor und/oder 3 Chloratomen, oder durch Hydroxyalkoxy, Hydroxyalkyl oder Cyanoalkyl mit jeweils bis zu 8 Kohlenstoffatomen, oder durch Alkenyloxy mit bis zu 6 Kohlenstoffatomen, oder durch eine Gruppe $-XR^3$,

wobei

X - eine geradkettige oder verzweigte Alkylen-oder Alkenylenkette mit bis zu 8 Kohlenstoffatomen bedeutet,

und

$R^3$ - Aryl mit 6 bis 10 Kohlenstoffatomen bedeutet, wobei der Arylrest bis zu 4 gleiche oder verschiedene Substituenten der Reihe Fluor, Chlor, Brom, Iod, Cyano, Alkyl, Alkoxy, Alkylthio mit jeweils bis zu 6 Kohlenstoffatomen, Nitro, Dioxymethylen, Dioxyethylen, Trifluormethyl, Trifluormethoxy, Difluormethoxy oder Trifluormethylthio tragen kann, oder

- einen 5- bis 6-gliedrigen gesättigten oder ungesättigten heterocyclischen Ring bedeutet, der benzokondensiert sein kann und der als Heteroatome Sauerstoff, Schwefel oder bis zu 2 Stickstoffatome enthalten kann, oder

- Hydroxy, Carboxy, Alkoxycarbonyl mit bis zu 6 Kohlenstoffatomen, Alkylcarbonyloxy mit bis zu 18 Kohlenstoffatomen, Cycloalkyl mit 3 bis 6 Kohlenstoffatomen bedeutet, oder
- eine Gruppe $-OSO_3H$, $-CONH-NH_2$, $-CONH_2$ oder Amino, Alkylamino oder Dialkylamino mit jeweils bis zu 6 Kohlenstoffatomen je Alkylgruppe bedeutet, oder
- Alkyl oder Hydroxyalkyl mit bis zu 8 Kohlenstoffatomen bedeutet,

oder

- für einen gesättigten, verbrückten Heterocyclus

oder

$R^1$ und $R^2$ gemeinsam mit dem Stickstoffatom einen 5- bis 6-gliedrigen Ring bilden, der anelliert sein kann, der durch Sauerstoff, Schwefel oder die Gruppe $-NR^4$ unterbrochen sein kann, und der substituiert sein kann durch Alkyl mit bis zu 4 Kohlenstoffatomen, Hydroxy, Phenyl, Carboxy oder Alkoxycarbonyl mit bis zu 4 Kohlenstoffatomen,

wobei

$R^4$ - Aryl mit 6 bis 10 Kohlenstoffatomen bedeutet, wobei der Arylrest bis 4 gleiche oder verschiedene Substituenten der Gruppe Fluor, Chlor, Brom, Iod, Cyano, Nitro, Hydroxy, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder

- Alkoxycarbonyl mit bis zu 6 Kohlenstoffatomen bedeutet, oder
- Pyridyl, Pyrimidyl, Furyl, Thienyl, Pyrazinyl, Pyridazinyl oder Chinolyl bedeutet, oder
- Cycloalkyl mit 3 bis 7 Kohlenstoffatomen bedeutet, oder
- Alkyl oder Alkenyl mit bis zu 6 Kohlenstoffatomen bedeutet, das bis zu 2-fach gleich oder verschieden substituiert sein kann durch gegebenenfalls durch Chlor, Dioxymethylen oder Trifluormethyl substituiertes Phenyl, Hydroxy, Amino, Alkylamino, Dialkylamino mit jeweils bis zu 3 Kohlenstoffatomen je Alkylgruppe, Fluor, Chlor, Brom, Cycloalkyl mit 3 bis 6 Kohlenstoffatomen, durch Carboxy oder Alkoxycarbonyl mit bis zu 6 Kohlenstoffatomen, durch Pyridyl, Pyrimidyl, Pyrazinyl, Morpholinyl, Thiomorpholinyl, Pyrrolidinyl oder Pyrrolindinocarbonyl, durch Morpholinocarbonyl, durch Alkylaminocarbonyl, Dialkylaminocarbonyl oder Phenylalkylaminocarbonyl mit jeweils bis zu 6 Kohlenstoffatomen je Alkylgruppe substituiert sein kann.

Bevorzugt seien Verbindungen der allgemeinen Formel (I) genannt, in welcher

n - für die Zahl 1 bis 4 steht,

$R^1$ - für Wasserstoff steht,

und

$R^2$ - für geradkettiges oder verzweigtes Alkyl mit bis zu 6 Kohlenstoffatomen steht, das substituiert sein kann durch Pyridyl, Thienyl oder durch Phenyl, wobei der Phenylrest bis zu 3 gleiche oder verschiedene Substituenten der Reihe Fluor, Chlor, Brom, Hydroxy, Alkyl, Alkoxy mit bis zu 4 Kohlenstoffatomen, Dioxymethylen, Dioxyethylen oder Carboxy, oder

- für Cyclopentyl, Cyclohexyl oder Cycloheptyl steht,

oder

- für Chinuclidin steht

oder

- für 1,2,3,4-Tetrahydroisochinolin steht,
- für Phenyl steht, das bis zu 3-fach gleich oder verschieden substituiert sein kann durch Fluor, Chlor, Brom, Cyano, Alkyl mit bis zu 4 Kohlenstoffatomen, Hydroxyalkyl oder Hydroxyalkoxy mit bis zu 4 Kohlenstoffatomen, Trifluormethyl, Trifluormethoxy, Dioxymethylen, Dioxyethylen, durch gegebenenfalls im Aromaten durch Fluor, Chlor, Brom, Hydroxy, Alkyl, Alkoxy mit bis zu 9 Kohlenstoffatomen, Dioxymethylen, Trifluormethyl oder Carboxy substituiertes Phenoxy, oder durch geradkettiges oder verzweigtes Alkenyloxy mit bis zu 4 Kohlenstoffatomen, oder durch eine Gruppe $-XR^3$

wobei

X - eine geradkettige oder verzweigte Alkylenkette mit bis zu 6 Kohlenstoffatomen bedeutet

und

$R^3$ - ein bis zu 3-fach gleich oder verschieden durch Fluor, Chlor, Brom, Hydroxy, Alkyl, Alkoxy mit bis zu 4 Kohlenstoffatomen, Dioxymethylen, Trifluormethyl oder Carboxy substituiertem Phenyl bedeutet, oder

- Pyridyl, Morpholinyl oder Piperidinyl, bedeutet, oder
- Hydroxy, Carboxy, Alkoxycarbonyl mit bis zu 4 Kohlenstoffatomen, oder Cyclopropyl, Cyclopentyl oder Cyclohexyl bedeutet, oder
- Amino, Alkylamino oder Dialkylamino mit jeweils bis zu 4 Kohlenstoffatomen je Alkylgruppe bedeutet,

oder

$R^1$ und $R^2$ gemeinsam mit dem Stickstoff einen Ring der Formel

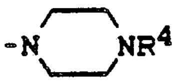, 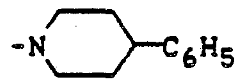, 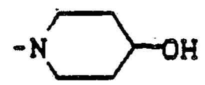, 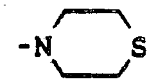

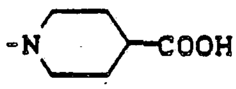 oder 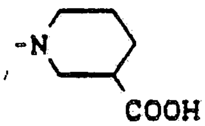 bilden,

wobei
$R^4$ - einen gegebenenfalls bis zu 3-fach gleich oder verschieden durch Fluor, Chlor, Brom, Hydroxy, Alkyl, Alkoxy mit bis zu 4 Kohlenstoffatomen, Dioxymethylen, Trifluormethyl oder Carboxy substituiertes Phenyl bedeutet, oder
- Alkoxycarbonyl mit bis zu 4 Kohlenstoffatomen bedeutet, oder
- Pyridyl oder Pyrimidyl bedeutet, oder
- Cyclopropyl, Cyclopentyl oder Cyclohexyl bedeutet, oder
- geradkettiges oder verzweigtes Alkyl oder Alkenyl mit bis zu 4 Kohlenstoffatomen bedeutet, das bis zu 2-fach gleich oder verschieden durch gegebenenfalls durch Chlor, Trifluormethyl oder Dioxymethylen substituiertes Phenyl, Hydroxy, Amino, Methylamino, Dimethylamino, Fluor, Chlor, Cyclopropyl, Cyclopentyl, Cyclohexyl oder Alkoxycarbonyl mit bis zu 4 Kohlenstoffatomen, durch Morpholinyl, Pyrrolidinyl oder Pyrrolidinocarbonyl Morpholinocarbonyl, Dialkylamino oder Phenylalkylamino mit jeweils bis zu 4 Kohlenstoffatomen substituiert sein kann.

Besonders bevorzugt seien Verbindungen der allgemeinen Formel (I) genannt, in welcher
n - für die Zahl 2 steht,
$R^1$ - für Wasserstoff steht,
und
$R^2$ - für geradkettiges oder verzweigtes Alkyl mit bis zu 3 Kohlenstoffatomen, das substituiert sein kann durch Thienyl, Phenyl,
- für Phenyl steht, das bis zu 3-fach gleich oder verschieden substituiert sein kann durch Fluor, Chlor, Brom, Cyano, Alkyl mit bis zu 4 Kohlenstoffatomen, Hydroxyalkyl mit bis zu 6 Kohlenstoffatomen, Alkoxy mit bis zu 9 Kohlenstoffatomen, Hydroxyalkoxy mti bis zu 4 Kohlenstoffatomen, Alkylthio mit bis zu 4 Kohlenstoffatomen, Trifluormethyl, Trifluormethoxy,
oder
- für Chinuclidinyl steht oder
- für 1,2,3,4-Tetrahydroisochinolin steht
$R^1$ und $R^2$ gemeinsam mit dem Stickstoff einen Ring der Formel

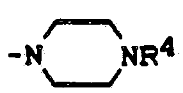 oder 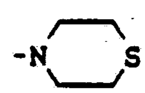 bilden,

wobei
$R^4$ - einen gegebenenfalls bis zu 2-fach gleich oder verschieden durch Fluor, Chlor, Brom, Hydroxy, Alkyl, Alkoxy mit bis zu 4 Kohlenstoffatomen, substituertes Phenyl bedeutet, oder
- Alkoxycarbonyl mit bis zu 4 Kohlenstoffatomen bedeutet, oder
- Cyclopropyl, Cyclopentyl oder Cyclohexyl bedeutet, oder
- geradkettiges oder verzweigtes Alkyl oder Alkenyl mit bis zu 4 Kohlenstoffatomen bedeutet, das bis zu 2-fach gleich oder verschieden durch gegebenenfalls durch Hydroxy, Amino, Dimethylamino, Cyclopropyl oder Alkoxycarbonyl mit bis zu 4 Kohlenstoffatomen, durch Morpholinyl, Pyrrolidinyl oder Pyrrolidinocarbonyl substituiert sein kann.

Ganz besonders bevorzugt seien folgende Verbindungen:
3-(1,5-Didesoxy-1,5-imino-D-glucit-N-yl)-propionsäure-(4-methoxy)-anilid,
3-(1,5-Didesoxy-1,5-imino-D-glucit-N-yl)-propionsäure-(4-methoxycarbonyl)-anilid,
3-(1,5-Didesoxy-1,5-imino-D-glucit-N-yl)-propionsäure-4-(4-pyridylmethoxy)-anilid,

3-(1,5-Didesoxy-1,5-imino-D-glucit-N-yl)-propionsäure-4-benzyloxy-anilid,
3-(1,5-Didesoxy-1,5-imino-D-glucit-N-yl)-propionsäure-4-trifluormethoxy-anilid,
3-(1,5-Didesoxy-1,5-imino-D-glucit-N-yl)-propionsäure-4-(2-oxocyclohexyloxy)-anilid,
3-(1,5-Didesoxy-1,5-imino-D-glucit-N-yl)-propionsäure-4-tert.butyl-anilid,
3-(1,5-Didesoxy-1,5-imino-D-glucit-N-yl)-propionsäure-4-(6-methyl-heptyloxy)-anilid,
3-(1,5-Didesoxy-1,5-imino-D-glucit-N-yl)-propionsäure-4-(2-N,N-diethyl-amino-ethoxy)-anilid,
3-(1,5-Didesoxy-1,5-imino-D-glucit-N-yl)-propionsäure-4-allyloxy-anilid,
3-(1,5-Didesoxy-1,5-imino-D-glucit-N-yl)-propionsäure-4-trifluormethyl-anilid,
3-(1,5-Didesoxy-1,5-imino-D-glucit-N-yl)-propionsäure-4-(2-chlor-1,1,2-trifluorethoxy)-anilid,
3-(1,5-Didesoxy-1,5-imino-D-glucit-N-yl)-propionsäure-(4-hydroxy)-anilid,
3-(1,5-Didesoxy-1,5-imino-D-glucit-N-yl)-propionsäure-4-isopropyl-anilid,
3-(1,5-Didesoxy-1,5-imino-D-glucit-N-yl)-propionsäure-2-isopropyl-anilid,
3-(1,5-Didesoxy-1,5-imino-D-glucit-N-yl)-propionsäure-4-fluor-anilid,
3-(1,5-Didesoxy-1,5-imino-D-glucit-N-yl)-propionsäure-2-fluor-anilid,
1-[3-(1,5-Didesoxy-1,5-imino-D-glucit-N-yl)-propionyl]-2,3-dihydroindol,
3-(1,5-Didesoxy-1,5-imino-D-glucit-N-yl)-propionsäure-2,4-difluoranilid,
3-(1,5-Didesoxy-1,5-imino-D-glucit-N-yl)-propionsäure-2,4-difluoranilid,
3-(1,5-Didesoxy-1,5-imino-D-glucit-N-yl)-propionsäure-2-trifluormethyl-anilid,
3-(1,5-Didesoxy-1,5-imino-D-glucit-N-yl)-propionsäure-2,6-difluoranilid,
3-(1,5-Didesoxy-1,5-imino-D-glucit-N-yl)-propionsäure-(adamantan-1-yl)-amid,
3-(1,5-Didesoxy-1,5-imino-D-glucit-N-yl)-propionsäure-3,4,5-trimethoxy-anilid,
3-(1,5-Didesoxy-1,5-imino-D-glucit-N-yl)-propionsäure-3,4-dimethoxy-anilid,
3-(1,5-Didesoxy-1,5-imino-D-glucit-N-yl)-propionsäure-3,5-dimethoxy-anilid,
3-(1,5-Didesoxy-1,5-imino-D-glucit-N-yl)-propionsäure-2,4,6-tribromanilid,
3-(1,5-Didesoxy-1,5-imino-D-glucit-N-yl)-propionsäure-3-cyano-anilid,
3-(1,5-Didesoxy-1,5-imino-D-glucit-N-yl)-propionsäure-4-cyclohexyl-anilid,
3-(1,5-Didesoxy-1,5-imino-D-glucit-N-yl)-propionsäure-3,4-ethylendioxy-anilid,
3-(1,5-Didesoxy-1,5-imino-D-glucit-N-yl)-propionsäure-2-bromanilid,
3-(1,5-Didesoxy-1,5-imino-D-glucit-N-yl)-propionsäure-3,4-methylendioxy-anilid,
3-(1,5-Didesoxy-1,5-imino-D-glucit-N-yl)-propionsäure-2-methylthio-anilid,
3-(1,5-Didesoxy-1,5-imino-D-glucit-N-yl)-propionsäure-2-methylthio-anilid,
3-(1,5-Didesoxy-1,5-imino-D-glucit-N-yl)-propionyl-4-(4-trifluormethyl-phenyl)-piperazin,
3-(1,5-Didesoxy-1,5-imino-D-glucit-N-yl)-propionsäure-anilid,
3-(1,5-Didesoxy-1,5-imino-D-glucit-N-yl)-propionsäure-4-ethoxy-anilid,
3-(1,5-Didesoxy-1,5-imino-D-glucit-N-yl)-propionsäure-2-dimethylaminoethyl-amid,
3-(1,5-Didesoxy-1,5-imino-D-glucit-N-yl)-propionsäure-[2-(3-methoxyphenyl)ethyl)-amid,
3-(1,5-Didesoxy-1,5-imino-D-glucit-N-yl)-propionsäure-2-pyridylmethyl-amid,
3-(1,5-Didesoxy-1,5-imino-D-glucit-N-yl)-propionyl-(4-cinnamyl)-piperazin,
3-(1,5-Didesoxy-1,5-imino-D-glucit-N-yl)-4-(2-hydroxyethoxy)-anilid,
3-(1,5-Didesoxy-1,5-imino-D-mannit-N-yl)-propionsäure-(4-methoxy)-anilid,
3-(1,5-Didesoxy-1,5-imino-D-mannit-N-yl)-propionsäure-(4-methoxycarbonyl)-anilid,
3-(1,5-Didesoxy-1,5-imino-D-mannit-N-yl)-propionsäure-4-(4-pyridylmethoxy)-anilid,
3-(1,5-Didesoxy-1,5-imino-D-mannit-N-yl)-propionsäure-4-benzyloxy-anilid,
3-(1,5-Didesoxy-1,5-imino-D-mannit-N-yl)-propionsäure-4-trifluormethoxy-anilid,
3-(1,5-Didesoxy-1,5-imino-D-mannit-N-yl)-propionsäure-4-(2-oxocyclohexyloxy)-anilid,
3-(1,5-Didesoxy-1,5-imino-D-mannit-N-yl)-propionsäure-4-tert.butyl-anilid,
3-(1,5-Didesoxy-1,5-imino-D-mannit-N-yl)-propionsäure-4-(6-methyl-heptyloxy)-anilid,
3-(1,5-Didesoxy-1,5-imino-D-mannit-N-yl)-propionsäure-4-(2-N,N-diethyl-amino-ethoxy)-anilid,
3-(1,5-Didesoxy-1,5-imino-D-mannit-N-yl)-propionsäure-4-allyloxy-anilid,
3-(1,5-Didesoxy-1,5-imino-D-mannit-N-yl)-propionsäure-4-trifluormethyl-anilid,
3-(1,5-Didesoxy-1,5-imino-D-mannit-N-yl)-propionsäure-4-(2-chlor-1,1,2-trifluorethoxy)-anilid,
3-(1,5-Didesoxy-1,5-imino-D-mannit-N-yl)-propionsäure-4-isopropyl-anilid,
3-(1,5-Didesoxy-1,5-imino-D-mannit-N-yl)-propionsäure-2-isopropyl-anilid,
3-(1,5-Didesoxy-1,5-imino-D-mannit-N-yl)-propionsäure-4-fluor-anilid,
3-(1,5-Didesoxy-1,5-imino-D-mannit-N-yl)-propionsäure-2-fluor-anilid,
1-[3-(1,5-Didesoxy-1,5-imino-D-mannit-N-yl)-propionyl]-2,3-dihydroindol,
3-(1,5-Didesoxy-1,5-imino-D-mannit-N-yl)-propionsäure-2,4-difluoranilid,
3-(1,5-Didesoxy-1,5-imino-D-mannit-N-yl)-propionsäure-2,4-difluoranilid,

3-(1,5-Didesoxy-1,5-imino-D-mannit-N-yl)-propionsäure-2-trifluormethyl-anilid,
3-(1,5-Didesoxy-1,5-imino-D-mannit-N-yl)-propionsäure-2,6-difluoranilid,
3-(1,5-Didesoxy-1,5-imino-D-mannit-N-yl)-propionsäure-(adamantan-1-yl)-amid,
3-(1,5-Didesoxy-1,5-imino-D-mannit-N-yl)-propionsäure-3,4,5-trimethoxy-anilid,
3-(1,5-Didesoxy-1,5-imino-D-mannit-N-yl)-propionsäure-3,4-dimethoxy-anilid,
3-(1,5-Didesoxy-1,5-imino-D-mannit-N-yl)-propionsäure-3,5-dimethoxy-anilid,
3-(1,5-Didesoxy-1,5-imino-D-mannit-N-yl)-propionsäure-3,5-dimethoxy-anilid,
3-(1,5-Didesoxy-1,5-imino-D-mannit-N-yl)-propionsäure-2,4,6-tribromanilid,
3-(1,5-Didesoxy-1,5-imino-D-mannit-N-yl)-propionsäure-3-cyano-anilid,
3-(1,5-Didesoxy-1,5-imino-D-mannit-N-yl)-propionsäure-4-cyclohexyl-anilid,
3-(1,5-Didesoxy-1,5-imino-D-mannit-N-yl)-propionsäure-3,4-ethylendioxy-anilid,
3-(1,5-Didesoxy-1,5-imino-D-mannit-N-yl)-propionsäure-2-bromanilid,
3-(1,5-Didesoxy-1,5-imino-D-mannit-N-yl)-propionsäure-3,4-methylendioxy-anilid,
3-(1,5-Didesoxy-1,5-imino-D-mannit-N-yl)-propionsäure-2-methylthio-anilid,
3-(1,5-Didesoxy-1,5-imino-D-mannit-N-yl)-propionsäure-2-methylthio-anilid,
3-(1,5-Didesoxy-1,5-imino-D-mannit-N-yl)-propionyl-4-(4-trifluormethyl-phenyl)-piperazin,
3-(1,5-Didesoxy-1,5-imino-D-mannit-N-yl)-propionsäure-anilid,
3-(1,5-Didesoxy-1,5-imino-D-mannit-N-yl)-propionsäure-4-ethoxy-anilid,
3-(1,5-Didesoxy-1,5-imino-D-mannit-N-yl)-propionsäure-2-dimethylaminoethyl-amid,
3-(1,5-Didesoxy-1,5-imino-D-mannit-N-yl)-propionsäure-[2-(3-methoxyphenyl)ethyl)-amid,
3-(1,5-Didesoxy-1,5-imino-D-mannit-N-yl)-propionsäure-2-pyridylmethyl-amid,
3-(1,5-Didesoxy-1,5-imino-D-mannit-N-yl)-propionyl-(4-cinnamyl)-piperazin und
3-(1,5-Didesoxy-1,5-imino-D-mannit-N-yl)-propionsäure-4-(2-hydroxyethoxy)-anilid,
3-(1,5-Didesoxy-1,5-imino-D-mannit-N-yl)-propionyl-1-(4-p-fluorphenyl)piperazin,
3-(1,5-Didesoxy-1,5-imino-D-mannit-N-yl)-propionitril
3-(1,5-Didesoxy-1,5-imino-D-mannit-N-yl)-propionsäure
1,5-Didexoxy-1,5-imino-D-mannit-N-yl)-propionyl-1-(4-phenyl)-piperazin
3-(1,5-Didesoxy-1,5-imino-D-mannit-N-yl)-propionyl-1(4-phenyl)-piperazin,
3-(1,5-Didesoxy-1,5-imino-D-glucit-N-yl)-propionyl-1-(4-cyclopropyl)-piperazin,
3-(1,5-Didesoxy-1,5-imino-D-glucit-N-yl)-propionsäure-(4-trifluormethyl-benzyl)-amid,
3-(1,5-Didesoxy-1,5-imino-D-glucit-N-yl)-propionsäure-(2-phenyl-2-Keto-ethyl)-amid,
3-(1,5-Didesoxy-1,5-imino-D-glucit-N-yl)-propionyl-1-(4-2-fluorphenyl)-piperazin,
3-(1,5-Didesoxy-1,5-imino-D-glucit-N-yl)-propionyl-1-(4-nitrophenyl)-piperazin,
3-(1,5-Didesoxy-1,5-imino-D-glucit-N-yl)-propionyl-1-(4-cyanophenyl)-piperazin,
3-(1,5-Didesoxy-1,5-imino-D-glucit-N-yl)-propionyl-1-(4-methylphenyl)-piperazin
3-(1,5-Didesoxy-1,5-imino-D-glucit-N-yl)-propionyl-1-(4-[3-trifluormethyl-4-chlorphenyl)-piperazin
3-(1,5-Didesoxy-1,5-imino-D-glucit-N-yl)-propionyl-1-(4-[morpholinoessigsäure-2-yl])-piperazin,
3-(1,5-Didesoxy-1,5-imino-D-glucit-N-yl-propionyl-1-(4-[2-pyrimidyl])-piperazin,
3-(1,5-Didesoxy-1,5-imino-D-glucit-N-yl)-propionyl-1-thiomorpholid,
3-(1,5-Didesoxy-1,5-imino-D-glucit-N-yl)-propionsäure-(thiophen-2-yl)-methylamid,
3-(1,5-Didesoxy-1,5-imino-D-glucit-N-yl)-propionsäure-(1S)-1-phenylethylamid,
3-(1,5-Didesoxy-1,5-imino-D-glucit-N-yl)-propionsäure-(1R)-phenylethylamid
3-(1,5-Didesoxy-1,5-imino-D-glucit-N-yl)-propionsäure-butylamid
3-(1,5-Didesoxy-1,5-imino-D-glucit-N-yl)-propionsäure-t-butylamid,
3-(1,5-Didesoxy-1,5-imino-D-glucit-N-yl)-propionsäure-4-decyloxyanilid,
4-(1,5-Didesoxy-1,5-imino-D-glucit-N-yl)-butansäure-4-ethoxy-anilid,
4-(1,5-Didesoxy-1,5-imino-D-glucit-N-yl)-butansäure-4-t-butyl-anilid
4-(1,5-Didesoxy-1,5-imino-D-glucit-N-yl)-butansäure-4-(2-chlor-1,1,2-trifluorethoxy)-anilid,
4-(1,5-Didesoxy-1,5-imino-D-glucit-N-yl)-butansäure-4-cyclohexyl-anilid,
1-[3-(1,5-Didesoxy-1,5-imino-D-glucit-N-yl)-propionyl]-1,2,3,4-tetrahydroisochinolin.

Im Rahmen von Untersuchungen die zu der vorliegenden Erfindung führten, wurde überraschenderweise gefunden, daß die erfindungsgemäßen Verbindungen der allgemeinen Formel (I) eine außerordentlich gute Wirkung gegen Retro-Viren besitzen.

Die erfindungsgemäßen Verbindungen können hergestellt werden, indem man
Carbonsäuren der allgemeinen Formel (II)

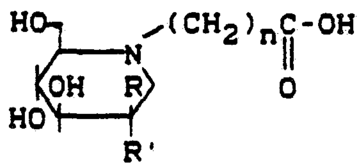

(II)

in welcher
n die oben angegebene Bedeutung hat,
nach bekannten Methoden, gegebenenfalls über ein reaktives Säurederivat, mit Aminen der allgemeinen Formel (III)

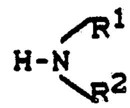

(III),

in welcher
$R^1$ und $R^2$ die oben angegebene Bedeutung haben,
gegebenenfalls in Anwesenheit eines inerten organischen Lösemittels umsetzt.

Als reaktive Säurederivate seien beispielsweise genannt: aktivierte Ester, Hydroxysuccinimidester, Säureimidazolide, Säurehalogenide, gemischte Anhydride, oder die Umsetzung in Anwesenheit von Carbodiimiden wie beispielsweise Cyclohexylcarbodiimid.

Das erfindungsgemäße Verfahren kann beispielsweise durch folgendes Formelschema erläutert werden:

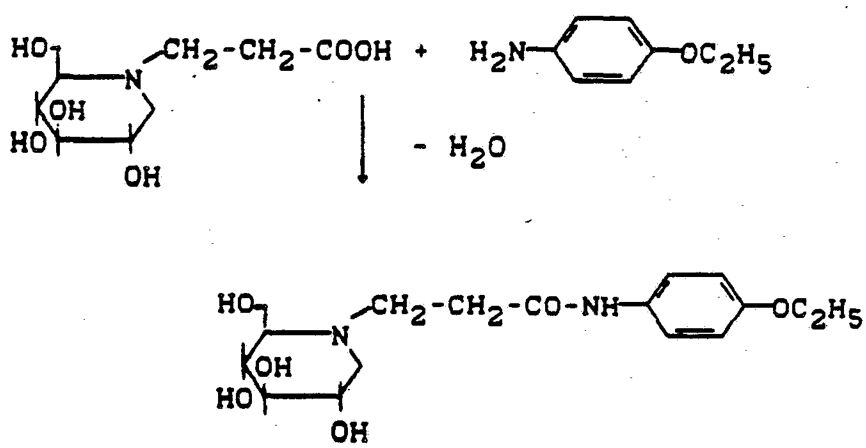


Die Durchführung des erfindungsgemäßen Verfahrens lehnt sich an die literaturbekannte Methodik zur Überführung von Carbonsäuren in Carbonsäureamide an. Dabei wird die Carbonsäure zunächst in eine aktivierte Form wie z.B. das Säurechlorid oder das Imidazolid überführt, die entweder als solche isoliert und in einem zweiten Reaktionsschritt umgesetzt werden, oder die in situ direkt zu den erfindungsgemäßen Verbindungen amidiert werden. Als aktivierende Reagenzien seien neben den anorganischen Halogeniden wie Thionylchlorid, Phosphortrichlorid oder Phosphorpentachlorid, oder dem Carbonyldiimidazol, Carbodiimide wie Dicyclohexylcarbodiimid oder 1-Cyclohexyl-3-[2 (N-methyl-morpholino)ethyl]carbodiimid-p-toluolsulfonat oder N-Hydroxyphthalimid oder N-Hydroxy-benztriazol in Gegenwart von Dicyclohexylcarbodiimid beispielhaft erwähnt. Naturgemäß lassen sich die Piperidin-N-yl-carbonsäuren auch in Form ihrer Salze einsetzen. [Die Methodik der Amidierung wird beispielsweise beschrieben: Fieser & Fieser, Reagents for Organic Synthesis, John Wiley & Sons Inc. (1967), Seite 231-236; J.C. Shihan and G.P. Hess, J. Am. Chem. Soc. 77, 1067 (1955); U. Goodman, G.W. Kenner, Adv. in Protein Chem. 12, 488 (1957); W.A. Bonner, P.I. McNamee, J. Org. Chem. 26, 254 (1961); H.A. Staab, Angew. Chemie Int. Ed. 1, 351 (1962); Fieser & Fieser, Reagents for Organic Synthesis, John Wiley & Sons Inc. 1967, 116, 114; H.C. Beyerman, U.O. van der Brink, Re. Trav. 80, 1372 (1961); C.A. Buehler, D.E. Pearson, John Wiley & Sons, Volume I (1970),

Seite 895 ff, Volume II, (1977)].

Als Lösemittel für das erfindungsgemäße Verfahren kommen neben Wasser alle inerten organischen Lösemittel in Frage, die sich unter den Reaktionsbedingungen nicht verändern. Hierzu gehören vorzugsweise Ether wie Diethylether Dioxan, Tetrahydrofuran, Glykolmonomethylether oder Gly koldimethylether, oder halogenierte Kohlenwasserstoffe wie Dichlormethan, Trichlormethan oder Tetrachlormethan, oder Amide wie Dimethylformamid, Dimethylacetamid oder Hexamethylphosphorsäuretriamid, oder Kohlenwasserstoffe wie Benzol, Toluol oder Xylol, oder Acetonitril, Nitromethan, Pyridin, Dimethylsulfoxid oder Essigester. Ebenso können Gemische der genannten Lösemittel verwendet werden. Isoliert man die aktivierten Zwischenstufen der Dihydropyridinmonocarbonsäuren, so können auch die Amine der Formel (X) alleine als Verdünnungsmittel verwendet werden.

Die Reaktionstemperaturen können in einem breiten Bereich variiert werden. Im allgemeinen arbeitet man in einem Bereich von -70° C bis +140° C, bevorzugt von -20° C bis +100° C.

Die Umsetzung kann bei Normaldruck, aber auch bei erhöhtem oder erniedrigtem Druck durchgeführt werden. Im allgemeinen arbeitet man bei Normaldruck.

Bei der Durchführung des erfindungsgemäßen Verfahrens ist das Verhältnis der an der Reaktion beteiligten Stoffe beliebig. Im allgemeinen arbeitet man mit molaren Mengen der Reaktanden. Es hat sich jedoch als günstig erwiesen, das Amin in einem 5- bis 10-fach molaren Überschuß einzusetzen. Besonders zweckmäßig wird das Amin in einem großen Überschuß direkt als Lösemittel eingesetzt.

Die als Ausgangsstoffe eingesetzten D-gluco konfigurierten Carbonsäuren von 1-Desoxy-nojirimycin sind bekannt [DE 30 24 901; DE 27 58 025; DE 29 22760; JP 81/103 153].

Die als Ausgangsstoffe eingesetzten D-manno konfigurierten Carbonsäuren von 1-Desoxy-nojirimycin und ihre Herstellung sind neu.

Als Carbonsäuren werden beispielsweise erfindungsgemäß verwendet:

N-[2-Carboxyethyl]-1-desoxy-nojirimycin,
N-[3-Carboxypropyl]-1-desoxy-nojirimycin,
N-[4-Carboxybutyl]-1-desoxy-nojirimycin,
N-[2-Carboxyethyl]-1-desoxy-manno-nojirimycin,
N-[3-Carboxypropyl]-1-desoxy-manno-nojirimycin oder
N-[4-Carboxybutyl]-1-desoxy-manno-nojirimycin.

Die als Ausgangsstoffe eingesetzten Amine sind bekannt oder können nach bekannten Methoden hergestellt werden wie sie z.B. in Beilstein's Handbuch der Organischen Chemie beschrieben werden.

Als Amine werden beispielsweise erfindungsgemäß verwendet:

2-Fluoranilin,
4-Fluoranilin,
2-Bromanilin,
4-Iodanilin,
2-Trifluormethyl-anilin,
4-Hydroxy-anilin,
3-Trifluormethyl-anilin,
4-Trifluormethyl-anilin,
3-Cyan-anilin,
2-Methylmercapto-anilin,
3,4-Methylendioxy-anilin,
3,4-Ethylendioxy-anilin,
3,4-Dimethoxy-anilin,
3,5-Dimethoxy-anilin,
3,4,5-Trimethoxy-anilin,
2,6-Difluoranilin,
2,4-Difluoranilin,
3,4-Difluoranilin,
2,4,6-Tribromanilin,
2-Isopropyl-anilin,
4-Isopropyl-anilin,
4-(t-Butyl)-anilin,
4-Cyclohexyl-anilin,
2,3-Dihydroindol,
1,2,3,4-Tetrahydroisochinolin,
Thiomorpholin,

2,2-Dimethyl-1-propylamin,
2-Ethyl-butylamin,
Cyclopentylamin,
Cycloheptylamin,
1-Phenyl-piperazin,
1-(2-Methylphenyl)-piperazin,
1-(3-Methylphenyl)-piperazin,
1-(4-Methylphenyl)-piperazin,
1-(2-Ethylphenyl)-piperazin,
1-(2-Chlorphenyl)-piperazin,
1-(3-Chlorphenyl)-piperazin,
1-(2-Fluorphenyl)-piperazin,
1-(4-Fluorphenyl)-piperazin,
1-(2-Cyanophenyl)-piperazin,
1-(2-Nitrophenyl)-piperazin,
1-(4-Nitrophenyl)-piperazin,
1-(2-Methoxyphenyl)-piperazin,
1-(3-Methoxyphenyl)-piperazin,
1-(4-Methoxyphenyl)-piperazin,
1-(2-Ethoxyphenyl)-piperazin,
1-(2-Methylmercaptophenyl)-piperazin,
1-(2-Hydroxyphenyl)-piperazin,
1-(4-Hydroxyphenyl)-piperazin,
1-(4-Trifluormethylphenyl)-piperazin,
1-(3-Trifluormethylphenyl)-piperazin,
1-(3-Trifluormethyl-4-chlorphenyl)-piperazin,
1-(2,3-Dimethylphenyl)-piperazin,
1-(2,6-Dimethylphenyl)-piperazin,
1-(3,4-Dimethylphenyl)-piperazin,
1-(3,5-Dichlorphenyl)-piperazin,
1-(3,4-Methylendioxyphenyl)-piperazin,
1-(2,4-Dimethoxyphenyl)-piperazin,
1-(3,4-Dimethoxyphenyl)-piperazin,
1-(3,5-Dimethoxyphenyl)-piperazin,
1-(3,4,5-Trimethoxyphenyl)-piperazin,
1-(2-Pyridyl)-piperazin,
1-(4-Pyridyl)-piperazin,
1-(2-Pyrimidyl)-piperazin,
1-(2-Pyrazinyl)-piperazin,
1-Benzyl-piperazin,
Piperzin-N-carbonsäureethylester,
1-(3,4-Methylendioxybenzyl)-piperazin,
1-(4-Chlor-benzyl)-piperazin,
1-(2-Phenylethyl)-piperazin,
1-(1-Phenylethyl)-piperazin,
1-Benzhydrylpiperazin,
1-(4,4′-Difluor-benzhydryl)-piperazin,
1-Cinnamyl-piperazin,
1-Allyl-piperazin,
1-Isopropyl-piperazin,
1-Cyclopropyl-piperazin,
1-Cyclobutyl-piperazin,
1-Cyclopentyl-piperazin,
1-Cyclohexyl-piperazin,
1-Cycloheptyl-piperazin,
1-(2-Cyclohexyl-ethyl)-piperazin,
1-(2-Dimethylamino-ethyl)-piperazin,
1-(2-Diethylamino-ethyl)-piperazin,

1-(3-Hydroxypropyl)-piperazin,
1-(3-Chorpropyl)-piperazin,
Piperazino-essigsäuremorpholid,
Piperazino-essigsäurepyrrolidid,
Piperazino-essigsäure-N-methyl-anilid,
Piperazino-essigsäureethylester,
4-Phenyl-piperidin,
4-Hydroxy-piperidin,
Piperidin-4-carbonsäure,
Piperidin-3-carbonsäure,
4-Fluorbenzylamin,
2-Chlorbenzylamin,
3-Chlorbenzylamin,
4-Brombenzylamin,
2-Methyl-benzylamin,
4-(t-Butyl)-benzylamin,
2-Methoxy-benzylamin,
3-Methoxy-benzylamin,
4-Methoxy-benzylamin,
3,4-Methylendioxy-benzylamin,
3,4-Dimethoxy-benzylamin,
3,4,5-Trimethoxy-benzylamin,
4-Trifluormethyl-benzylamin,
4-Carboxy-benzylamin,
3,5-Dimethyl-benzylamin,
D(+)-1-Phenylethylamin,
L(-)-1-Phenyl-ethylamin,
2-Aminomethyl-pyridin,
2-Aminomethyl-thiophen,
2-Cyclohexylsulfonylanilin,
2-(3,4-Dichlorbenzylsulfonyl)-anilin,
2-(3,4-Dichlorbenzylsulfonyl)-5-acetylamino-anilin,
2-(3,4-Dichlorbenzylsulfonyl)-4-acetylamino-anilin,
2-(Phenylsulfonyl)-4-acetylamino-anilin,
2-(3,4-Dichlorbenzylsulfonyl)-4-methyl-anilin,
4-(2-Hydroxy-ethoxy)-anilin,
4-(Cyclohexylmethoxy)-anilin,
(5-Ethylsulfonyl-1,3,4-thiadiazol-2-yl)-amin,
(5,6-Dihydro-4H-1,3-oxazin-2-yl)-amin,
2-Amino-4-trifluormethyl-pyrimidin,
2-Hydrazino-4-trifluormethyl-pyrimidin,
4,5-Bistrifluormethyl-2-hydrazino-pyrimidin,
4,6-Bistrifluormethyl-2-hydrazino-pyrimidin,
3,4-Bistrifluormethyl-anilin,
3,5-Bistrifluormethyl-anilin,
2-(2-Methoxycarbonyl-phenylthio)-5-methyl-anilin,
4-Methoxy-anilin
2,6-Dichlor-4-isobutoxycarbonyl-anilin,
4-Isobutoxycarbonyl-anilin,
2-Amino-5-dimethylamino-1,3,4-thiadiazol,
1-Amino-4-hydroxymethyl-piperidin,
6-Amino-5-chlor-8-methoxy-2-methyl-chinolin,
4-(5-Methylhexyloxy)-anilin oder
4-(Pyridylmethoxy)-anilin
4-Ethoxy-anilin.

Es wurde im Rahmen von Untersuchungen, die zu der vorliegenden Erfindung führten, überraschend gefunden, daß die erfindungsgemäßen Verbindungen der allgemeinen Formel (I) eine außerordentlich starke Wirkung gegen Viren besitzen und zwar sowohl gegen DNS-Viren als auch gegen RNS-Viren. Zu den DNS-Viren gehören z.B. die Piconda-, Papova-, Adeno-, Herpes-, Hepatitis- und Pockenviren. Zu der Gruppe der RNS-Viren gehören z.B. die Picorna-,. Reo, Toga-, Corona-, Othomyxo- und Rhabdoviren. Besonders wirksam sind die erfindungsgemäßen Verbindungen gegen die zu den RNS-Viren gehörenden Retroviren. Dies wird durch die weiter unten angegebenen Versuchsdaten beispielhaft für die Verbindungen am Visna-Virus in Zellkulturen belegt. Das Visna-Virus und das HIV-Virus (Virus der humanen Immundefiziens) gehören beide zu der Retrovirus-Subfamilie der Lentiviren [Haase A.T. Nature (1986) 322, 130-136]. Beide Viren weisen eine ähnliche Genomorganisation und ein gegenüber den anderen Retroviren komplexes Transkriptionsmuster auf [Sonijo P. et al., Cell (1985), 42, 369 - 382; Davis J. L. et al., Journal of Virology (1987) 61 (5) 1325 -1331].

In Zellkulturen, die mit Visna-Virus infiziert sind, treten 5 bis 10 Tage nach der Infektion ausgeprägte virusinduzierte, zytopathische Effekte auf. Durch Behandlung der infizierten Zellkulturen mit den erfindungsgemäßen Verbindungen konnte das Auftreten dieser zytopathischen Effekte verhindert werden.

Der Visna-Virus-Test wurde nach der Methode von O. Narayan et al., Journal of Infektious Diseases 135, 5, 1977, 800-806 durchgeführt. Dazu wurden die erfindungsgemäßen Verbindungen im Kulturmedium in nicht zytotoxischen Konzentrationen in 96er Microtiterplatten verdünnt. Anschließend wurden Fibroblastenzellen vom Schaf ($5 \times 10^4$ Zellen pro Napf) in Produktionsmedium zu jedem Näpfchen zugeführt. Jedes Näpfchen erhielt dann 50 µl einer Visna-Viruslösung mit einem Titer von ca. $2,5 \times 10^4$ $TCID_{50}$ (TCID = tissue culture infections dosis). Diese Virusdosis entspricht einer MOI (Multiplizität der Infektion) von ca. 0,05.

Unter diesen Infektionsbedingungen resultierte zwischen Tag 5 und Tag 10 in einer Infektionskontrolle ohne Substanz ein virusinduzierter, zytopathischer Effekt. Die infizierten und behandelten Zellen und die Kontrollzellen wurden 7 Tage bei 37° C 5% $CO_2$ inkubiert.

Bei Auftreten des virusinduzierten zytopathogenen Effektes in der unbehandelten Viruskontrolle wurden die Kulturen mit Formalin fixiert und anschließend mit einer Giemsa-Lösung gefärbt. Die inhibitorische Konzentration ($IC_{50}$) wurde mikroskopisch als die Konzentration ermittelt, bei der der zytopathische Effekt um 50% gehemmt wurde: im Vergleich zur unbehandelten Viruskontrolle, die 100% Zellzerstörung aufwies.

Es wurde gefunden, daß beispielsweise 3-(1,5-Didesoxy-1,5-imino-D-glucit-N-yl)-propionsäure-4-ethoxyanilid, in einem Konzentrationsbereich von 500 µg/ml bis zu 15 µg/ml die mit Visna-Virus infizierten Zellen vor der virusinduzierten Zellzerstörung schützen.

Typische Wirkung, wie sie für die erfindungsgemäßen Verbindungen aufgefunden werden, sind in Tabelle 1 aufgelistet. Darin bedeuten MCC Zytotoxizität und MIC die minimale Konzentration, bei der noch eine Inhibition der Virusvermehrung beobachtet wurde.

Tabelle 1

| Beisp. Nr. | 1 MCC µg/ml | 2 MIC µg/ml | Beisp. Nr. | 1 MCC µg/ml | 2 MIC µg/ml |
|---|---|---|---|---|---|
| 4 | >1000 | 125 | 60 | >1000 | 500 |
| 7 | >1000 | 500 | 66 | 1000 | 500 |
| 10 | >1000 | 30 | 68 | 500 | 250 |
| 11 | >1000 | 500 | 72 | >1000 | 500 |
| 12 | 1000 | 250 | 76 | >1000. | 500 |
| 14 | >1000 | 125 | 82 | >1000 | 500 |
| 16 | >1000 | 250 | 87 | >1000 | 250 |
| 17 | 1000 | 125 | 89 | 1000 | 500 |
| 18 | >1000 | 500 | 90 | 1000 | 250 |
| 19 | >1000 | 125 | 92 | >1000 | 250 |
| 23 | 150 | 75 | 93 | >1000 | 500 |
| 24 | >1000 | 125 | 94 | >1000 | 500 |
| 25 | >1000 | 250 | 96 | >1000 | 500 |
| 27 | >1000 | 250 | 99 | >1000 | 125 |
| 28 | >1000 | 500 | 00 | >1000 | 500 |
| 29 | >1000 | 125 | 89 | 1000 | 500 |
| 31 | >1000 | 125 | 90 | 1000 | 250 |
| 32 | >1000 | 250 | 92 | >1000 | 250 |
| 34 | >1000 | 15 | 93 | >1000 | 500 |
| 35 | 250 | 15 | 94 | >1000 | 500 |
| 38 | 1000 | 250 | 96 | >1000 | 500 |
| 40 | >1000 | 15 | 99 | >1000 | 125 |
| 41 | 1000 | 125 | 100 | >1000 | 500 |
| 43 | >1000 | 500 | 101 | 1000 | 125 |
| 50 | 1000 | 250 | 102 | >1000 | 125 |
| 51 | 1000 | 250 | 103 | >1000 | 125 |
| 53 | 500 | 250 | 104 | >1000 | 250 |
| 54 | 1000 | 250 | 107 | >1000 | 500 |
| 55 | 1000 | 250 | | | |

Da sie das Glykosylierungsmuster der Virusproteine verändern, können die erfindungsgemäßen Verbindungen auch bei der Herstellung abgeschwächter oder nicht infektiöser Viruspartikel verwendet werden, die ihrerseits zur Vakzineherstellung eingesetzt werden können.

Die erfindungsgemäß zu verwendenden Verbindungen stellen somit wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung oder Prophylaxe von menschlichen Retrovirusinfektionen.

2.) Für die Behandlung oder Prophylaxe von HIV (Virus der humanen Immundefiziens; früher HTLV III/LAV genannt) verursachten Erkrankungen (AIDS) und den damit assozierten Stadien wie ARC (AIDS related complex) und LAS (Lymphadennopathie-Syndrom) sowie der durch dieses Virus verursachten Immunschwäche und Encephalopathie.

3.) Für die Behandlung oder die Prophylaxe einer HTLV I-oder HTLV II-Infektion.

4.) Für die Behandlung oder die Prophylaxe des AIDS-carrier Zustandes (AIDS-Überträger-Zustand).

Als Indikationen in der Tiermedizin können beispielsweise angeführt werden:
Infektionen mit

a) Maedivisna (bei Schafen und Ziegen)

b) progressives Pneumonievirus (PPV) (bei Schafen und Ziegen

c) caprine arthritis encephalitis Virus (bei Schafen und Ziegen

d) Zwoegerziekte Virus (bei Schafen)

e) infektiöses Virus der Anämie (des Pferdes)

f) Infektionen verursacht durch das Katzenleukämievirus

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die Wirkstoffe der Formel (I) sollen in den oben aufgeführten pharmazeutischen Zubereitungen

vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungs gemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäß zu verwendenden Verbindungen können in den üblichen Konzentrationen und Zubereitungen zusammen mit dem Futter bzw. mit Futterzubereitungen oder mit dem Trinkwasser gegeben werden.

## Herstellungsbeispiele

### Beispiel 1

3-(1,5-Didesoxy-1,5-imino-D-glucit-N-yl)-propionsäure-(4-methoxy)-anilid

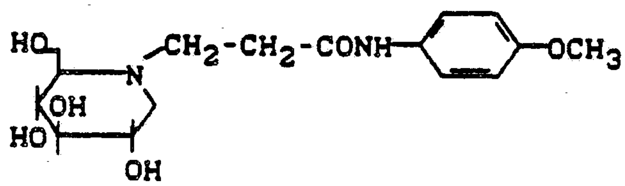

Man löst 2 g (8,5 mmol) N-(2-Carboxyethyl)-1-desoxynojirimycin in einer Mischung aus 11 ml Wasser und 22 ml Pyridin. Die Lösung wird auf 0° C abgekühlt. Eine Lösung von 1,9 g (9,4 mmol) Dicyclohexylcarbodiimid in Pyridin wird zugegeben und die Mischung wird 120 Min. bei 0° C belassen. Sodann werden 1,3 g (10,2 mmol) p-Methoxy-anilin zugesetzt. Die Kühlung wird entfernt und das Reaktionsgemenge bei Raumtemperatur gerührt. Nach 8 h wird dünnschichtchromatographisch (System Chloroform : Methanol :

Ammoniaklösung = 4:3:1 v/v) weitgehende Umsetzung detektiert.
Man filtriert den Festkörper ab, verdampft das Lösemittel im Hochvakuum und chromatographiert den Rückstand an 200 g Kieselgel (Mobile Phase Methylenchlorid : Ethanol : Triethylamin = 4:1:0.005).
Es werden 2,5 g (7,2 mmol, 85%) Produkt isoliert.
$R_f$ = 0,60 (Chloroform : Methanol : Ammonaik = 4:3:1)

Beispiel 2

3-(1,5-Didesoxy-1,5-imino-D-glucit-N-yl)-propionsäure-(4-oxymethylen-methoxycarbonyl)-anilid

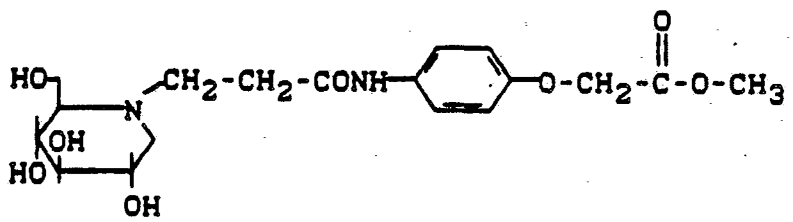

Entsprechend der Vorschrift aus Beispiel 1 werden 8,5 mmol N-(2-Carboxyethyl)-desoxynojirimycin mit 10 mmol 4-Oxymethylen-methoxycarbonyl-aniline.
Ausbeute: 1,45 g
Schmp.: 103° C $R_f$ = 0,60
$\alpha_D^{20}$ = -36°
(C = 1, Methanol)

Beispiel 3

3-(1,5-Didesoxy-1,5-imino-D-glucit-N-yl)-propionsäure-4-(4-pyridylmethoxy)-anilid

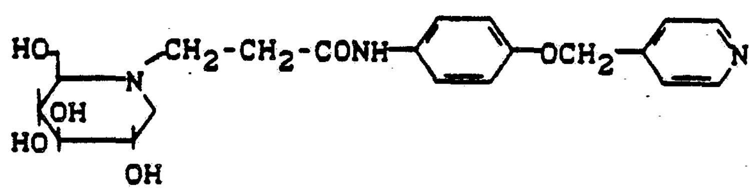

Entsprechend der Vorschrift aus Beispiel 1 werden 8,5 mmol N-(2-Carboxyethyl)-desoxynojirimycin mit 10 mmol 4-(4-Pyridylmethoxy)-anilin umgesetzt.
Ausbeute: 0,86 g
Schmp.: 172° C $R_f$ = 0,6
$\alpha_D^{20}$ = -38° (C=1, Methanol)

Beispiel 4

3-(1,5-Didesoxy-1,5-imino-D-glucit-N-yl)-propionsäure-4-benzyloxy-anilid

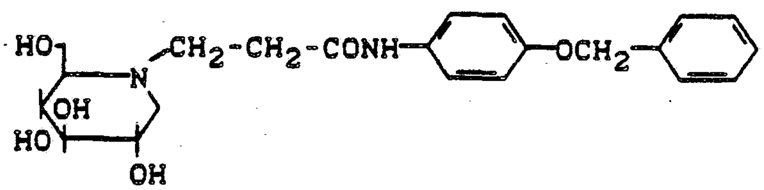

Entsprechend der Vorschrift aus Beispiel 1 werden 8,5 mmol N-(2-Carboxyethyl)-desoxynojirimycin mit 10 mmol 4-Benzyloxy-anilin umgesetzt.
Ausbeute: 1,26 g
Schmp.: 124° C  $R_f$ = 0,68
$\alpha_D^{20}$ = -25° (C=1, Methanol)

Beispiel 5

3-(1,5-Didesoxy-1,5-imino-D-glucit-N-yl)-propionsäure-4-trifluormethoxy-anilid

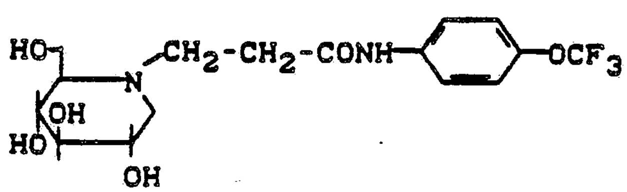

Entsprechend der Vorschrift aus Beispiel 1 werden 8,5 mmol N-(2-Carboxyethyl)-desoxynojirimycin mit 10 mmol 4-Trifluormethoxy-anilin umgesetzt.
$R_f$ = 0,64

Beispiel 6

3-(1,5-Didesoxy-1,5-imino-D-glucit-N-yl)-propionsäure-4-hydroxy-anilid

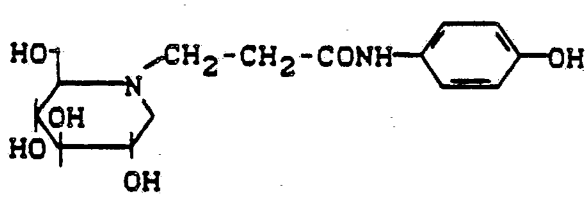

Entsprechend der Vorschrift aus Beispiel 1 werden 8,5 mmol N-(2-Carboxyethyl)-desoxynojirimycin mit 10 mmol 4-Hydroxy-anilin umgesetzt.
$R_f$ = 0,65
$\alpha_D^{20}$ = -25° (C=1, Methanol)

Beispiel 7

3-(1,5-Didesoxy-1,5-imino-D-glucit-N-yl)-propionsäure-4-tert.butyl-anilid

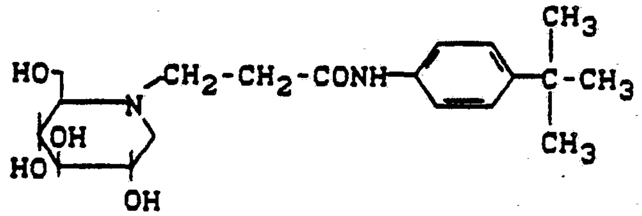

Entsprechend der Vorschrift aus Beispiel 1 werden 8,5 mmol N-(2-Carboxyethyl)-desoxynojirimycin mit 10 mmol 4-tert.-butyl-anilin umgesetzt.
Ausbeute: 1,97 g
Schmp.: 76° C $R_f$ = 0.68
$\alpha_D^{20}$ = -35° (C=1, Methanol)

Beispiel 8

3-(1,5-Didesoxy-1,5-imino-D-glucit-N-yl)-propionsäure-4-(6-methyl-heptyloxy)-anilid

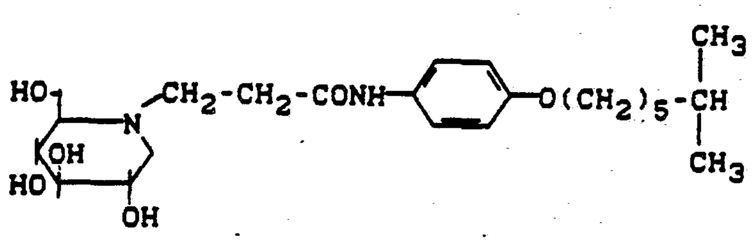

Entsprechend der Vorschrift aus Beispiel 1 werden 8,5 mmol N-(2-Carboxyethyl)-desoxynojirimycin mit 10 mmol 4-(6-Methyl-heptyloxy)-anilin umgesetzt.
Ausbeute: 1,39 g
Schmp.: fbl. Harz $R_f$ = 0,72
$\alpha_D^{20}$ = -29° (C=1, Methanol)

Beispiel 9

3-(1,5-Didesoxy-1,5-imino-D-glucit-N-yl)-propionsäure-4-(2-N,N-diethylamino-ethoxy)-anilid

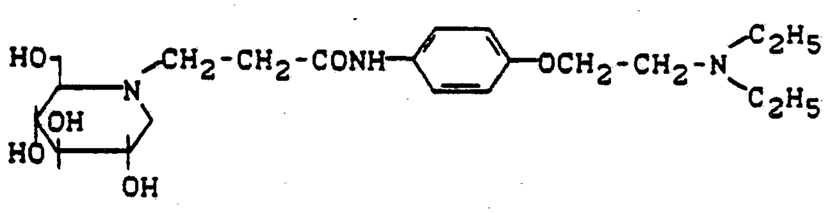

Entsprechend der Vorschrift aus Beispiel 1 werden 8,5 mmol N-(2-Carboxyethyl)-desoxynojirimycin mit 10 mmol 4-(2-N,N-diethylamino-ethoxy)-anilin umgesetzt.
Ausbeute: 1,44 g
Schmp.: fbl. Harz $R_f$ = 0,67
$\alpha_D^{20}$ = -28° (C=1, Methanol)

Beispiel 10

3-(1,5-Didesoxy-1,5-imino-D-glucit-N-yl)-propionsäure-4-allyloxy-anilid

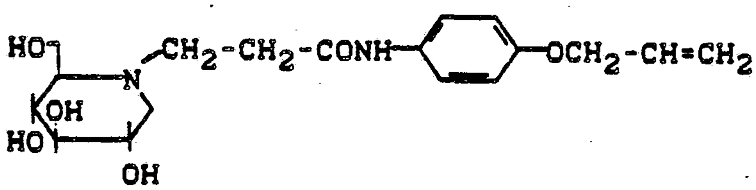

Entsprechend der Vorschrift aus Beispiel 1 werden 8,5 mmol N-(2-Carboxyethyl)-desoxynojirimycin mit 10 mmol 4-Allyloxy-anilin umgesetzt.
Ausbeute: 1,0
Schmp.: 139 °C $R_f$ = 0,66
$\alpha_D^{20}$ = -36° (C=1, Methanol)

Beispiel 11

3-(1,5-Didesoxy-1,5-imino-D-glucit-N-yl)-propionsäure-4-trifluormethyl-anilid

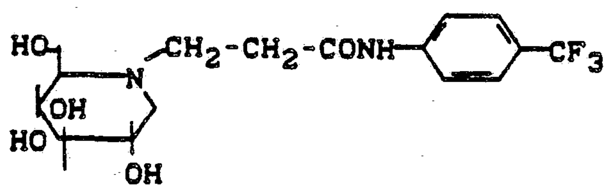

Entsprechend der Vorschrift aus Beispiel 1 werden 8,5 mmol N-(2-Carboxyethyl)-desoxynojirimycin mit 10 mmol 4-Trifluormethyl-anilin umgesetzt.
Ausbeute: 0,72 g
Schmp.: fbl. Harz $R_f$ = 0,65
$\alpha_D^{20}$ = - 24° (C=1, Methanol)

Beispiel 12

3-(1,5-Didesoxy-1,5-imino-D-glucit-N-yl)-propionsäure-4-(2-chlor-1,1,2-trifluorethoxy)-anilid

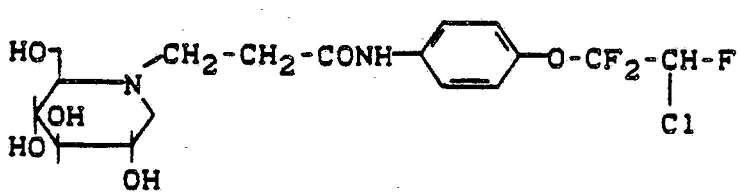

Entsprechend der Vorschrift aus Beispiel 1 werden 8,5 mmol N-(2-Carboxyethyl)-desoxynojirimycin mit 10 mmol 4-(2-chlor-1,1,2-trifluorethoxy)-anilin umgesetzt.

Ausbeute: 1,36 g
Schmp.: fbl. Harz  $R_f$ = 0,64
$\alpha_D^{20}$ = -27° (C=1, Methanol)

Beispiel 13

3-(1,5-Didesoxy-1,5-imino-D-glucit-N-yl)-propionsäure-4-isopropyl-anilid

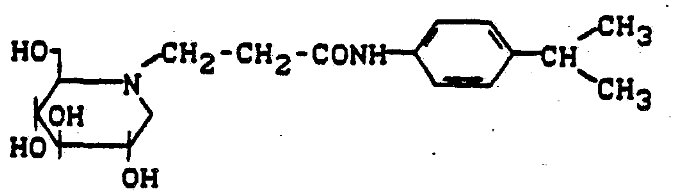

Entsprechend der Vorschrift aus Beispiel 1 werden 8,5 mmol N-(2-Carboxyethyl)-desoxynojirimycin mit 10 mmol 4-Isopropyl-anilin umgesetz.
Ausbeute: 1,7 g
Schmp.: fbl. Harz  $R_f$ = 0,64
$\alpha_D^{20}$ = -38° (C=1, Methanol)

Beispiel 14

3-(1,5-Didesoxy-1,5-imino-D-glucit-N-yl)-propionsäure-2-isopropyl-anilid

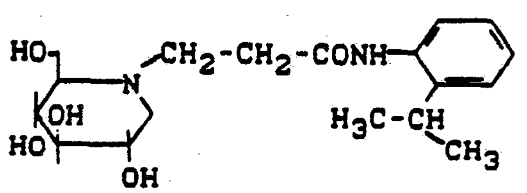

Entsprechend der Vorschrift aus Beispiel 1 werden 8,5 mmol N-(2-Carboxyethyl)-desoxynojirimycin mit 10 mmol 2-Isopropyl-anilin umgesetzt.
Ausbeute: 1,05 g
Schmp.: 156°C  $R_f$ = 0,68
$\alpha_D^{20}$ = -32° (C=1, Methanol)

Beispiel 15

3-(1,5-Didesoxy-1,5-imino-D-glucit-N-yl)-propionsäure-4-fluor-anilid

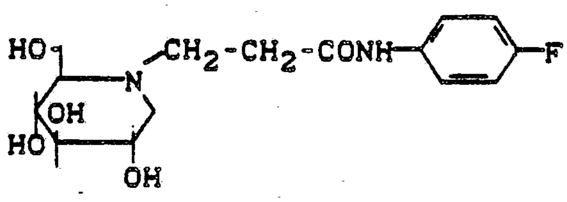

Entsprechend der Vorschrift aus Beispiel 1 werden 8,5 mmol N-(2-Carboxyethyl)-desoxynojirimycin mit 10 mmol 4-Fluoranilin umgesetzt.
Ausbeute: 1,7 g
Schmp.: fbl. Harz $R_f$ = 0,58
$\alpha_D^{20}$ = -31° (C=1, Methanol)

Beispiel 16

3-(1,5-Didesoxy-1,5-imino-D-glucit-N-yl)-propionsäure-2-fluor-anilid

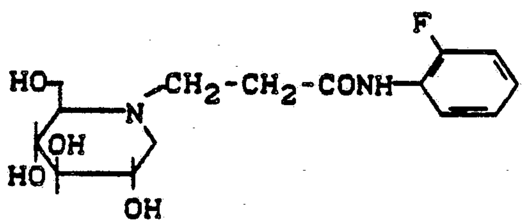

Entsprechend der Vorschrift aus Beispiel 1 werden 8,5 mmol N-(2-Carboxyethyl)-desoxynojirimycin mit 10 mmol 2-Fluoranilin umgesetzt.
Ausbeute: 0,92 g
Schmp.: 169°C $R_f$ = 0,66
$\alpha_D^{20}$ = -23° (C=1, Methanol))

Beispiel 17

3-(1,5-Didesoxy-1,5-imino-D-glucit-N-yl)-propionyl]-2,3-dihydroindol

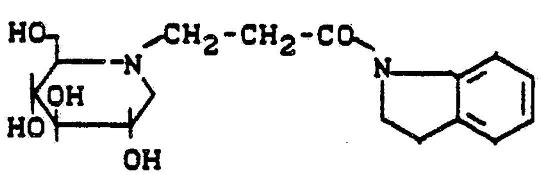

Entsprechend der Vorschrift aus Beispiel 1 werden 8,5 mmol N-(2-Carboxyethyl)-desoxynojirimycin mit 10 mmol 2,3-Dihydroindol umgesetzt.
Ausbeute: 1,2 g
Schmp.: 198°C $R_f$ = 0,7
$\alpha_D^{20}$ = -42° (C=1, Methanol)

Beispiel 18

3-(1,5-Didesoxy-1,5-imino-D-glucit-N-yl)-propionsäure-2,4-difluoranilid

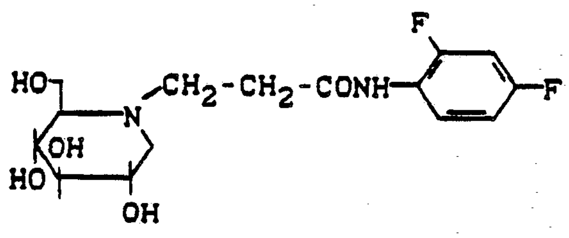

Entsprechend der Vorschrift aus Beispiel 1 werden 8,5 mmol N-(2-Carboxyethyl)-desoxynojirimycin mit 10 mmol 2,4-Difluoranilin umgesetzt.
Ausbeute: 1,03 g
Schmp.: 166 °C $R_f$ = 0,65
$\alpha_D^{20}$ = -24° (C=1, Methanol)

Beispiel 19

3-(1,5-Didesoxy-1,5-imino-D-glucit-N-yl)-propionsäure-3,4-difluoranilid

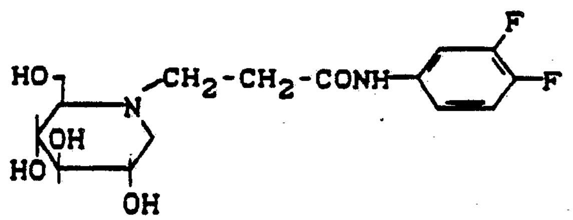

Entsprechend der Vorschrift aus Beispiel 1 werden 8,5 mmol N-(2-Carboxyethyl)-desoxynojirimycin mit 10 mmol 3,4-Difluoranilin umgesetzt.
Ausbeute: 1,33 g
Schmp.: fbl. Harz $R_f$ = 0,64
$\alpha_D^{20}$ = -26° (C=1, Methanol)

Beispiel 20

3-(1,5-Didesoxy-1,5-imino-D-glucit-N-yl)-propionsäure-2-trifluormethyl-anilid

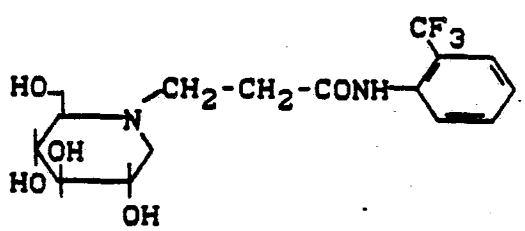

Entsprechend der Vorschrift aus Beispiel 1 werden 8,5 mmol N-(2-Carboxyethyl)-desoxynojirimycin mit

10 mmol 2-Trifluormethyl-anilin umgesetzt.
Ausbeute:
$R_f$ = 0,62

Beispiel 21

3-(1,5-Didesoxy-1,5-imino-D-glucit-N-yl)-propionsäure-2,6-difluoranilid

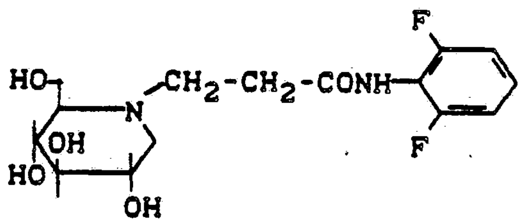

Entsprechend der Vorschrift aus Beispiel 1 werden 8,5 mmol N-(2-Carboxyethyl)-desoxynojirimycin mit 10 mmol 2,6-Difluoranilin umgesetzt.
$R_f$ = 0,62

Beispiel 22

3-(1,5-Didesoxy-1,5-imino-D-glucit-N-yl)-propionsäure-(adamantan-1-yl)-amid

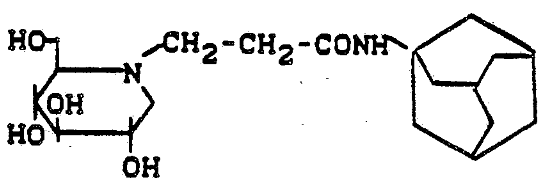

Entsprechend der Vorschrift aus Beispiel 1 werden 8,5 mmol N-(2-Carboxyethyl)-desoxynojirimycin mit 10 mmol 1-Amino-adamantan umgesetzt.
$R_f$ = 0,62

Beispiel 23

3-(1,5-Didesoxy-1,5-imino-D-glucit-N-yl)-propionsäure-3,4,5-trimethoxy-anilid

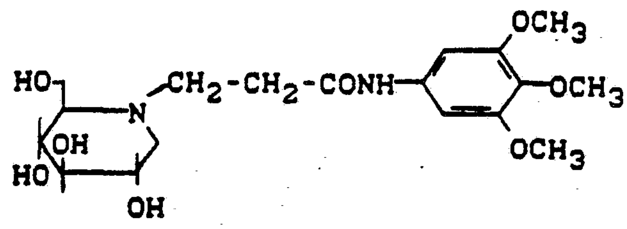

Entsprechend der Vorschrift aus Beispiel 1 werden 8,5 mmol N-(2-Carboxyethyl)-desoxynojirimycin mit 10 mmol 3,4,5-Trimethoxy-anilin umgesetzt.
Ausbeute: 2,16 g
Schmp.: fbl. Harz $R_f$ = 0,68
$\alpha_D^{20}$ = -29° (C=1, Methanol)

Beispiel 24

3-(1,5-Didesoxy-1,5-imino-D-glucit-N-yl)-propionsäure-3,4-dimethoxy-anilid

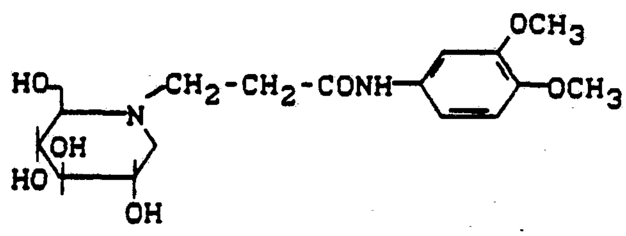

Entsprechend der Vorschrift aus Beispiel 1 werden 8,5 mmol N-(2-Carboxyethyl)-desoxynojirimycin mit 10 mmol 3,4-Dimethoxy-anilin umgesetzt.
Ausbeute: 1,78 g
Schmp.: 157° C $R_f$ = 0,65
$\alpha_D^{20}$ = -43° (C=1, Methanol)

Beispiel 25

3-(1,5-Didesoxy-1,5-imino-D-glucit-N-yl)-propionsäure-3,5-dimethoxy-anilid

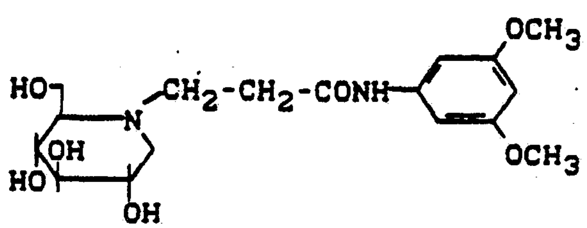

Entsprechend der Vorschrift aus Beispiel 1 werden 8,5 mmol N-(2-Carboxyethyl)-desoxynojirimycin mit 10 mmol 3,5-Dimethoxy-anilin umgesetzt.
Ausbeute: 1,82 g
Schmp.: 106° C $R_f$ = 0,68

$\alpha_D^{20}$ = -35° (C = 1, Methanol)

Beispiel 26

3-(1,5-Didesoxy-1,5-imino-D-glucit-N-yl)-propionsäure-2,4,6-tribromanilid

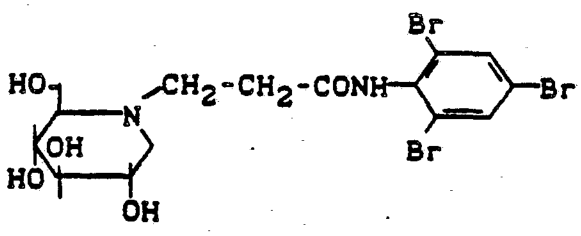

Entsprechend der Vorschrift aus Beispiel 1 werden 8,5 mmol N-(2-Carboxyethyl)-desoxynojirimycin mit 10 mmol 2,4,6-Tribromanilin umgesetzt.
Ausbeute: 0,98 g
Schmp.: 194° C    $R_f$ = 0,62
$\alpha_D^{20}$ = -16° (C = 1, Methanol)

Beispiel 27

3-(1,5-Didesoxy-1,5-imino-D-glucit-N-yl)-propionsäure-3-cyano-anilid

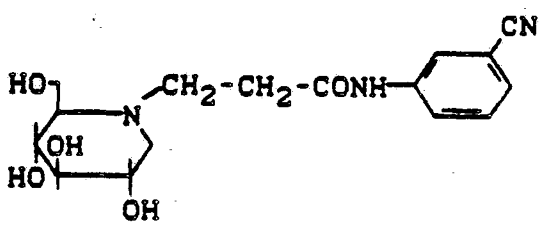

Entsprechend der Vorschrift aus Beispiel 1 werden 8,5 mmol N-(2-Carboxyethyl)-desoxynojirimycin mit 10 mmol 3-Cyano-anilin umgesetzt.
Ausbeute: 0,86
Schmp.: 177° C    $R_f$ = 0,64
$\alpha_D^{20}$ = -22° (C = 1, Methanol)

Beispiel 28

3-(1,5-Didesoxy-1,5-imino-D-glucit-N-yl)-propionsäure-4-cyclohexyl-anilid

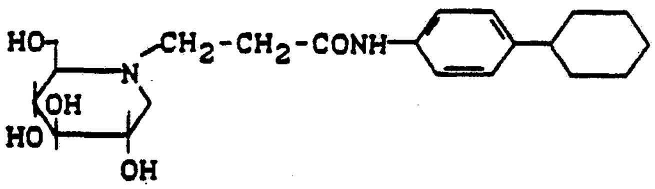

Entsprechend der Vorschrift aus Beispiel 1 werden 8,5 mmol N-(2-Carboxyethyl)-desoxynojirimycin mit 10 mmol 4-Cyclohexyl-anilin umgesetzt.
Ausbeute: 1,9 g
Schmp.: 206° C $R_f$ = 0,71
$\alpha_D^{20}$ = -34° (C=1, Methanol)

Beispiel 29

3-(1,5-Didesoxy-1,5-imino-D-glucit-N-yl)-propionsäure-3,4-ethylendioxy-anilid

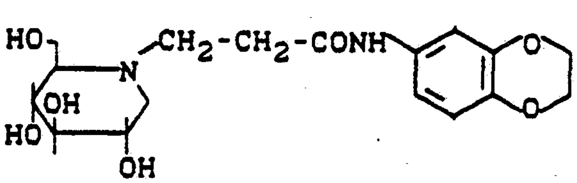

Entsprechend der Vorschrift aus Beispiel 1 werden 8,5 mmol N-(2-Carboxyethyl)-desoxynojirimycin mit 10 mmol 3,4-Ethylendioxy-anilin umgesetzt.
Ausbeute: 2,98 g
Schmp.: fbl. Harz $R_f$ = 0,62
$\alpha_D^{20}$ = -35° (C=1, Methanol)

Beispiel 30

3-(1,5-Didesoxy-1,5-imino-D-glucit-N-yl)-propionsäure-2-bromanilid

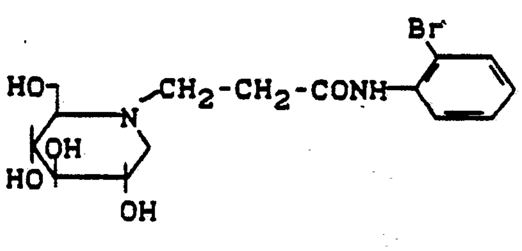

Entsprechend der Vorschrift aus Beispiel 1 werden 8,5 mmol N-(2-Carboxyethyl)-desoxynojirimycin mit 10 mmol 2-Bromanilin umgesetzt.
Ausbeute: 0,89 g
Schmp.: 201° C $R_f$ = 0,6
M+1 = 390

Beispiel 31

3-(1,5-Didesoxy-1,5-imino-D-glucit-N-yl)-propionsäure-3,4-methylendioxy-anilid

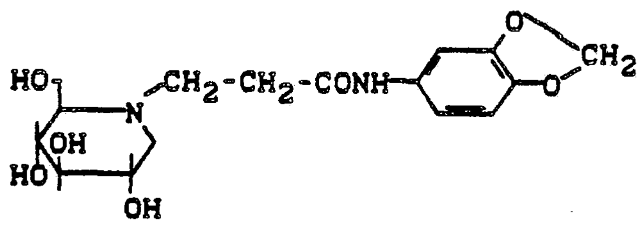

Entsprechend der Vorschrift aus Beispiel 1 werden 8,5 mmol N-(2-Carboxyethyl)-desoxynojirimycin mit 10 mmol 3,4-Methylendioxy-anilin umgesetzt.
Ausbeute: 1,59 g
Schmp.: fbl. Harz $R_f$ = 0,62
$\alpha_D^{20}$ = -37° (C=1, Methanol)

Beispiel 32

3-(1,5-Didesoxy-1,5-imino-D-glucit-N-yl)-propionsäure-2-methylthio-anilid

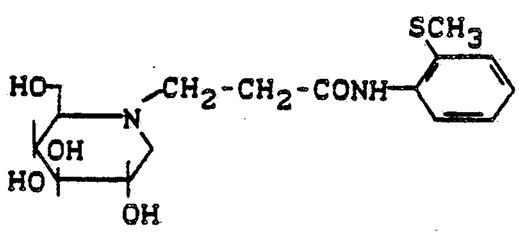

Entsprechend der Vorschrift aus Beispiel 1 werden 8,5 mmol N-(2-Carboxyethyl)-desoxynojirimycin mit 10 mmol 2-Methylthio-anilin umgesetzt.
Ausbeute: 0,8 g
Schmp.: 201 °C $R_f$ = 0,68

Beispiel 33

3-(1,5-Didesoxy-1,5-imino-D-glucit-N-yl)-propionyl-4-(4-trifluormethyl-phenyl)-piperazin

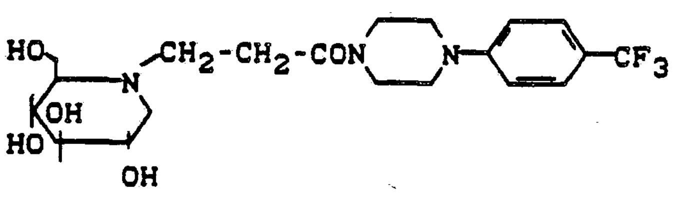

Entsprechend der Vorschrift aus Beispiel 1 werden 8,5 mmol N-(2-Carboxyethyl)-desoxynojirimycin mit 10 mmol 1-(4-Trifluormethylphenyl)-piperazin umgesetzt.
Ausbeute: 2,83 g
Schmp.: 179 °C    $R_f$ = 0,67
$\alpha_D^{20}$ = -11 ° (C = 1, Methanol)

Beispiel 34

3-(1,5-Didesoxy-1,5-imino-D-glucit-N-yl)-propionsäure-anilid

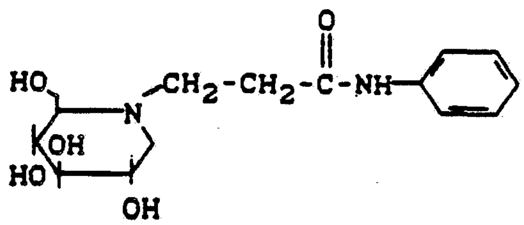

Entsprechend der Vorschrift aus Beispiel 1 werden 8,5 mmol N-(2-Carboxyethyl)-desoxynojirimycin mit 10 mmol Anilin umgeetzt.
Schmp.: 145 °C    $R_f$ = 0,52

Beispiel 35

3-(1,5-Didesoxy-1,5-imino-D-glucit-N-yl)-propionsäure-4-ethoxy-anilid

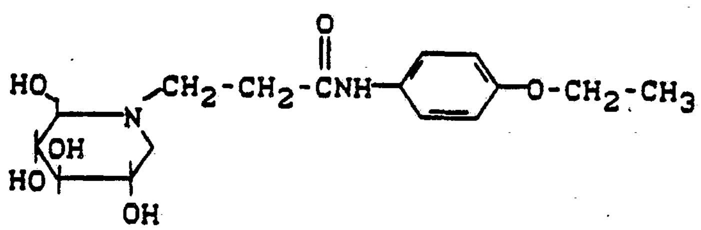

Entsprechend der Vorschrift aus Beispiel 1 werden 8,5 mmol N-(2-Carboxyethyl)-desoxynojirimycin mit 10 mmol 4-Ethoxy-anilin umgesetzt.
Schmp.: 195 °C    $R_f$ = 0,52

Beispiel 36

3-(1,5-Didesoxy-1,5-imino-D-glucit-N-yl)-propionsäure-2-dimethylaminoethyl-amid

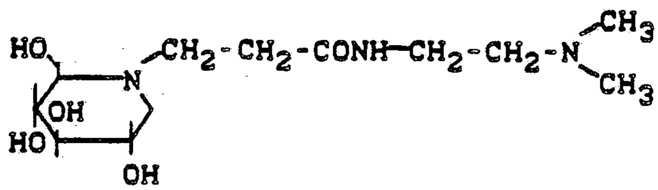

Entsprechend der Vorschrift aus Beispiel 1 werden 8,5 mmol N-(2-Carboxyethyl)-desoxynojirimycin mit 10 mmol 2-Dimethylaminoethylamin umgesetzt.
Ausbeute: 0,9 g
Schmp.: fbl. Harz    $R_f$ = 0,59
$\alpha_D^{20}$ = -2° (C=1, Methanol)

Beispiel 37

3-(1,5-Didesoxy-1,5-imino-D-glucit-N-yl)-propionsäure-[2-(3-methoxyphenyl)ethyl]amid

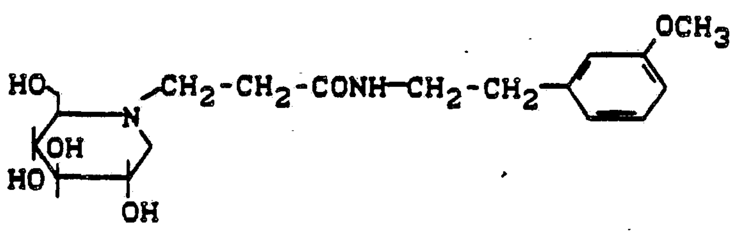

Entsprechend der Vorschrift aus Beispiel 1 werden 8,5 mmol N-(2-Carboxyethyl)-desoxynojirimycin mit 10 mmol 2-(3-Methoxyphenyl)ethylamin umgesetzt.
Ausbeute:
$R_f$ = 0,61

Beispiel 38

3-(1,5-Didesoxy-1,5-imino-D-glucit-N-yl)-propionsäure-2-pyridylmethyl-amid

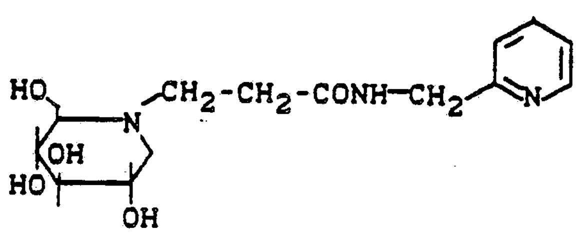

Entsprechend der Vorschrift aus Beispiel 1 werden 8,5 mmol N-(2-Carboxyethyl)-desoxynojirimycin mit 10 mmol 2-Aminomethyl-pyridin umgesetzt.
Ausbeute: 0,75 g
Schmp.: fbl. Harz    $R_f$ = 0,51
$\alpha_D^{20}$ = -15° (C=1, Methanol)

Beispiel 39

3-(1,5-Didesoxy-1,5-imino-D-glucit-N-yl)-propionyl-4-cinnamyl)-piperazin

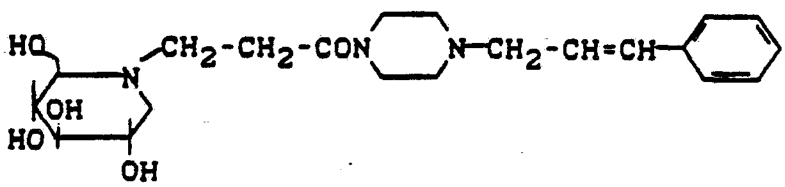

Entsprechend der Vorschrift aus Beispiel 1 werden 8,5 mmol N-(2-Carboxyethyl)-desoxynojirimycin mit 10 mmol 1-Cinnamyl-piperazin umgesetzt.
$R_f$ = 0,67

Beispiel 40

3-(1,5-Didesoxy-1,5-imino-D-glucit-N-yl)-4-(2-hydroxyethoxy)-anilid

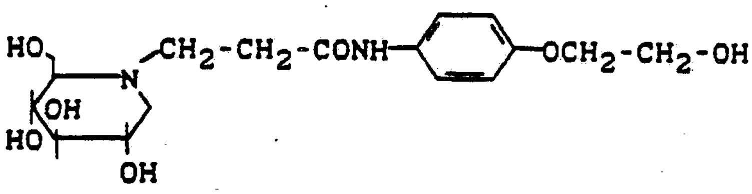

Entsprechend der Vorschrift aus Beispiel 1 werden 8,5 mmol N-(2-Carboxyethyl)-desoxynojirimycin mit 10 mmol 4-(2-Hydroxyethoxy)-anilin umgesetz.
Ausbeute:
Schmp.: 149 °C $R_f$ = 0,43

Beispiel 41

3-(1,5-Didesoxy-1,5-imino-D-glucit-N-yl)-propionyl-1-(4-p-fluorphenyl)-piperazin

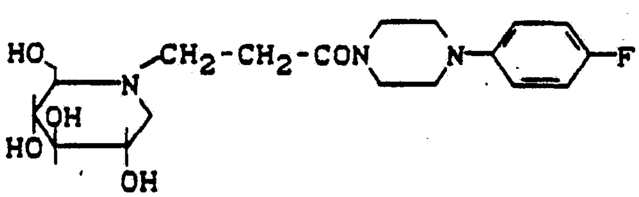

Entsprechend der Vorschrift aus Beispiel 1 werden 8,5 mmol N-(2-Carboxyethyl)-desoxynojirimycin mit 10 mmol 4-Fluorphenyl)-piperazin umgesetzt.
Ausbeute: 1,2 g
Schmp.: 155 °C $R_f$ = 0,64
$\alpha_D^{20}$ = -12° (C=1, Methanol)

Beispiel 42

3[(1,5-Didesoxy-1,5-imino-D-mannit-N-yl)-propionsäure-4-methoxy-anilid

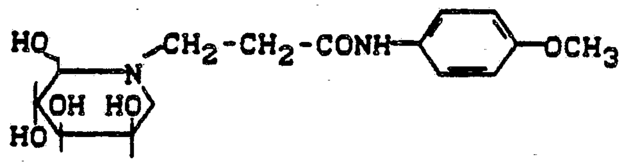

Man löst 2 g (8,5 mmol) N-(2-Carboxyethyl)-1-desoxymannonojirimycin in einer Mischung aus 11 ml Wasser und 22 ml Pyridin. Die Lösung wird auf 0° C abgekühlt. Eine Lösung von 1,9 g (9,4 mmol) Dicyclohexylcarbodiimid in Pyridin wird zugegeben und die Mischung wird 120 Min. bei 0° C belassen. Sodann werden 1,3 g (10,2 mmol) p-Methoxy-anilin zugesetzt. Die Kühlung wird entfernt und das Reaktionsgemenge bei Raumtemperatur gerührt.
Nach 8 h wird dünnschichtchromatographisch (System Chloroform : Methanol : Ammoniaklösung = 4:3:1 v/v) weitgehende Umsetzung detektiert.
Man filtriert den Festkörper ab, verdampft das Lösemittel im Hochvakuum und chromatographiert den Rückstand an 200 g Kieselgel (Mobile Phase Methylenchlorid : Ethanol : Triethylamin = 4:1:0.005).
Es werden 1,9 g Produkt isoliert.
$R_f$ = 0,60 (Chloroform : Methanol : Ammoniak = 4:3:1)

Beispiel 43

3-(1,5-Didesoxy-1,5-imino-D-mannit-N-yl)-propionsäure-(4-oxymethylen-methoxycarbonyl)-anilid

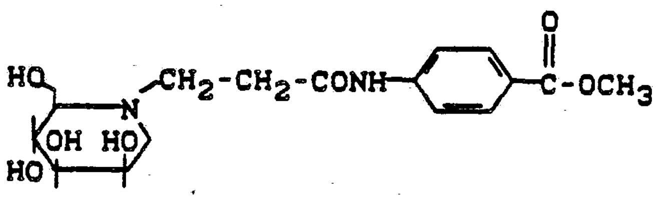

Entsprechend der Vorschrift aus Beispiel 1 werden 8,5 mmol N-(2-Carboxyethyl)-1-desoxy-mannonojirimycin mit 10 mmol 4- Methoxycarbonyl-anilin umgesetzt.
$R_f$ = 0,60
$\alpha_D^{20}$ = -27° (C = 1, Methanol)

Beispiel 44

3-(1,5-Didesoxy-1,5-imino-D-mannit-N-yl)-propionsäure-4-(4-pyridylmethoxy)-anilid

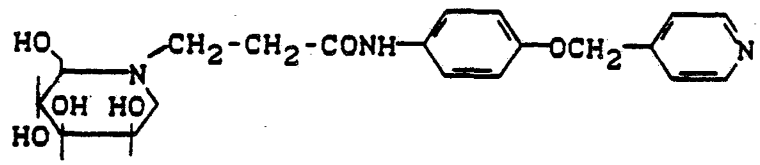

Beispiel 43

3-(1,5-Didesoxy-1,5-imino-D-mannit-N-yl)-propionsäure-(4-methoxycarbonyl)-anilid

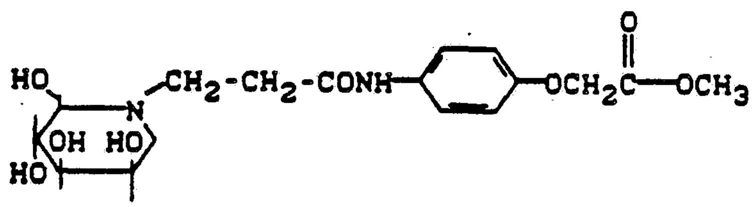

Entsprechend der Vorschrift aus Beispiel 1 werden 8,5 mmol N-(2-Carboxyethyl)-1-desoxy-mannonojirimycin mit 10 mmol 4- Methoxycarbonyl-1 anilin umgesetzt.
$R_f$ = 0,60
$\alpha_D^{20}$ = -27° (C=1, Methanol)

Beispiel 44

3-(1,5-Didesoxy-1,5-imino-D-mannit-N-yl)-propionsäure-4-(4-pyridylmethoxy)-anilid

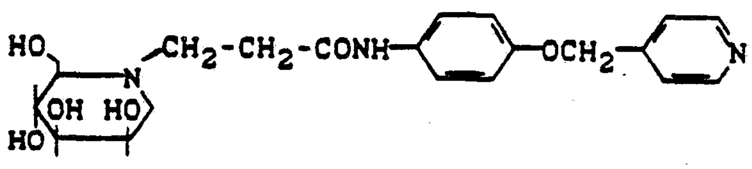

Entsprechend der Vorschrift aus Beispiel 1 werden 8,5 mmol N-(2-Carboxyethyl)-1-desoxy-mannonojirimycin mit 10 mmol 4-(4- Pyridylmethoxy)-anilin umgesetzt.
$R_f$ = 0,6

Beispiel 45

3-(1,5-Didesoxy-1,5-imino-D-mannit-N-yl)-propionsäure-4-benzyloxy-anilid

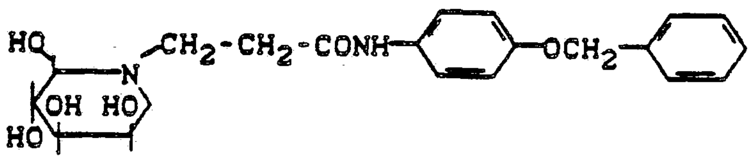

Entsprechend der Vorschrift aus Beispiel 1 werden 8,5 mmol N-(2-Carboxyethyl)-1-desoxy-mannonojirimycin mit 10 mmol 4- Benzyloxy-anilin umgesetzt.
$R_f$ = 0,68 $\alpha_D^{20}$ = -42° (C=1, Methanol)

Beispiel 46

3-(1,5-Didesoxy-1,5-imino-D-mannit-N-yl)-propionsäure-4-trifluormethyl)-anilid

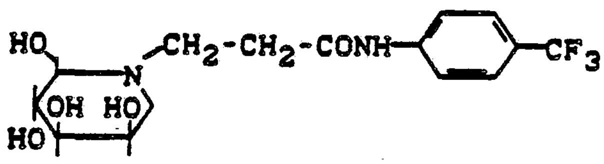

$\alpha_D^{20}$ = -38° (C=1, Methanol)
Entsprechend der Vorschrift aus Beispiel 1 werden 8,5 mmol N-(2-Carboxyethyl)-1-desoxy-mannonojirimycin mit 10 mmol 4- Trifluormethoxy-anilin umgesetzt.
$R_f$ = 0,64

Beispiel 47

3-(1,5-Didesoxy-1,5-imino-D-mannit-N-yl)-propionsäure-4-hydroxy-anilid

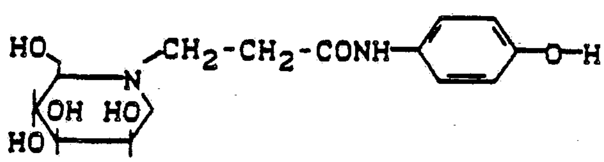

Entsprechend der Vorschrift aus Beispiel 1 werden 8,5 mmol N-(2-Carboxyethyl)-1-desoxy-mannonojirimycin mit 10 mmol 4-hydroxy-anilin umgesetzt.
$R_f$ = 0,65

Beispiel 48

3-(1,5-Didesoxy-1,5-imino-D-mannit-N-yl)-propionsäure-4-tert.butyl-anilid

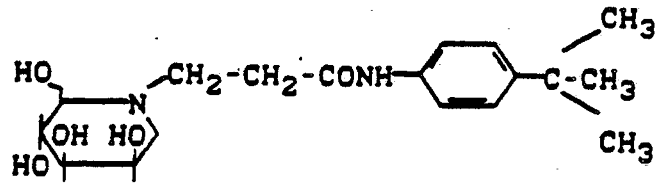

Entsprechend der Vorschrift aus Beispiel 1 werden 8,5 mmol N-(2-Carboxyethyl)-1-desoxy-mannonojirimycin mit 10 mmol 4-tert.butyl-anilin umgesetzt.
$R_f$ = 0,68
$\alpha_D^{20}$ = -39° (C = 1.0, Methanol)

Beispiel 49

3-(1,5-Didesoxy-1,5-imino-D-mannit-N-yl)-propionsäure-4-(6-methyl-heptyloxy)-anilid

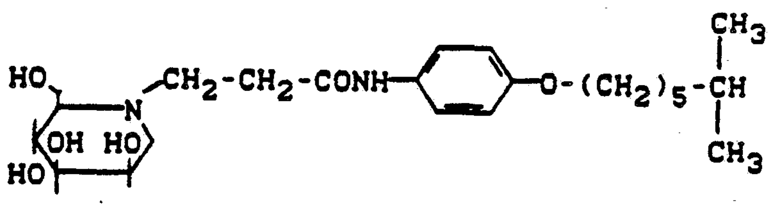

Entsprechend der Vorschrift aus Beispiel 1 werden 8,5 mmol N-(2-Carboxyethyl)-1-desoxy-mannonojirimycin mit 10 mmol 4-(6- Methyl-heptyloxy)-anilin umgesetzt.
$R_f$ = 0,72
$\alpha_D^{20}$ = -33° (C = 1.0, Methanol)

Beispiel 50

3-(1,5-Didesoxy-1,5-imino-D-mannit-N-yl)-propionsäure-4-(2-N,NN-diethylamino-ethoxy)-anilid

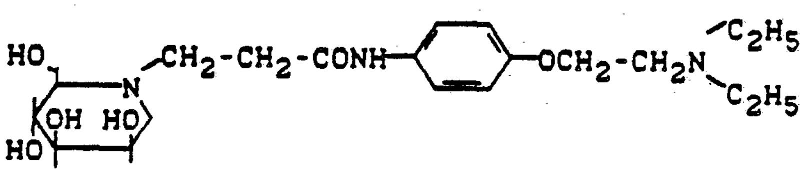

Entsprechend der Vorschrift aus Beispiel 1 werden 8,5 mmol N-(2-Carboxyethyl)-1-desoxy-mannonojirimycin mit 10 mmol 4-(2-N,N- diethylamino-ethoxy)-anilin umgesetzt.
$R_f$ = 0,67
$\alpha_D^{20}$ = -32° (C = 1.0, Methanol)

Beispiel 51

3-(1,5-Didesoxy-1,5-imino-D-mannit-N-yl)-propionsäure-4-allyloxy-anilid

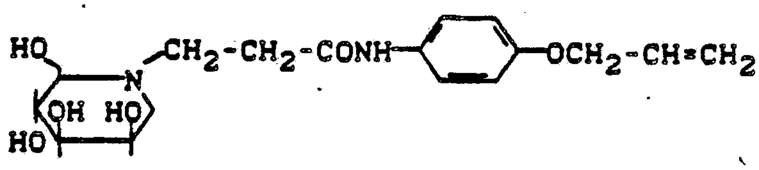

Entsprechend der Vorschrift aus Beispiel 1 werden 8,5 mmol N-(2-Carboxyethyl)-1-desoxy-mannonojirimycin mit 10 mmol 4- Allyloxy-anilin umgesetzt.
$R_f$ = 0,66
$\alpha_D^{20}$ = -45° (C = 1.0, Methanol)

Beispiel 52

3-(1,5-Didesoxy-1,5-imino-D-mannit-N-yl)-propionsäure-4-trifluormethyl-anilid

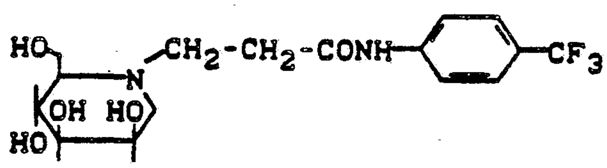

Entsprechend der Vorschrift aus Beispiel 1 werden 8,5 mmol N-(2-Carboxyethyl)-1-desoxy-mannonojirimycin mit 10 mmol 4- Trifluormethyl-anilin umgesetzt.
$R_f$ = 0,65
$\alpha_D^{20}$ = -38° (C = 1.0, Methanol)

Beispiel 53

3-(1,5-Didesoxy-1,5-imino-D-mannit-N-yl)-propionsäure-4-(2-chlor-1,1,2-trifluorethoxy)-anilid

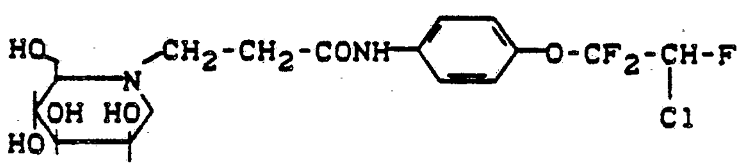

Entsprechend der Vorschrift aus Beispiel 1 werden 8,5 mmol N-(2-Carboxyethyl)-1-desoxy-mannonojirimycin mit 10 mmol 4-(2- chlor-1,1,2-trifluorethoxy)-anilin umgesetzt.
$R_f$ = 0,64
$\alpha_D^{20}$ = -38° (C = 1.0, Methanol)

Beispiel 54

3-(1,5-Didesoxy-1,5-imino-D-mannit-N-yl)-propionsäure-4-isopropyl-anilid

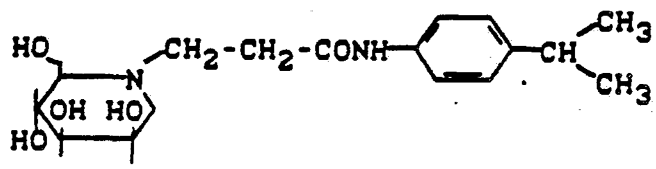

Entsprechend der Vorschrift aus Beispiel 1 werden 8,5 mmol N-(2-Carboxyethyl)-1-desoxy-mannonojirimycin mit 10 mmol 4- Isopropyl-anilin umgesetzt.
$R_f$ = 0,64
$\alpha_D^{20}$ = -41° (C=1.0, Methanol)

Beispiel 55

3-(1,5-Didesoxy-1,5-imino-D-mannit-N-yl)-propionsäure-2-isopropyl-anilid

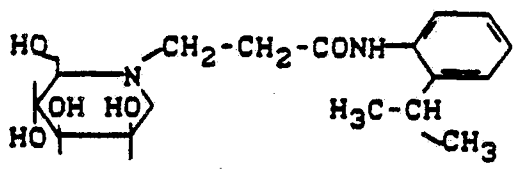

Entsprechend der Vorschrift aus Beispiel 1 werden 8,5 mmol N-(2-Carboxyethyl)-1-desoxy-mannonojirimycin mit 10 mmol 2- Isopropyl-anilin umgesetzt.
$R_f$ = 0.68
$\alpha_D^{20}$ = -36° (C=1.0, Methanol)

Beispiel 56

3-(1,5-Didesoxy-1,5-imino-D-mannit-N-yl)-propionsäure-4-fluor-anilid

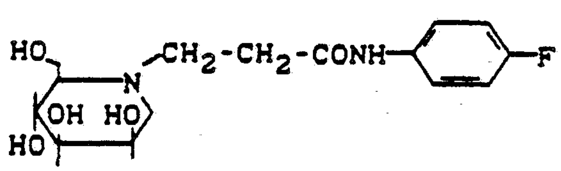

Entsprechend der Vorschrift aus Beispiel 1 werden 8,5 mmol N-(2-Carboxyethyl)-1-desoxy-mannonojirimycin mit 10 mmol 4-Fluor- anilin umgesetzt.
$R_f$ = 0,58
$\alpha_D^{20}$ = -42° (C=1.0, Methanol)

Beispiel 57

3-(1,5-Didesoxy-1,5-imino-D-mannit-N-yl)-propionsäure-2-fluor-anilid

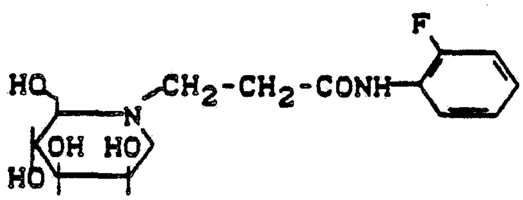

Entsprechend der Vorschrift aus Beispiel 1 werden 8,5 mmol N-(2-Carboxyethyl)-1-desoxy-mannonojirimycin mit 10 mmol 2-Fluor- anilin umgesetzt.
$R_f$ = 0,66
M+1 = 329

Beispiel 58

1-[3-(1,5-Didesoxy-1,5-imino-D-mannit-N-yl)-propionyl]-2,3-dihydroindol

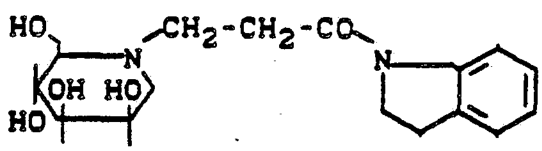

$R_f$ = 0,64
$\alpha_D^{20}$ = -43° (C=1, Methanol)
Entsprechend der Vorschrift aus Beispiel 1 werden 8,5 mmol N-(2-Carboxyethyl)-1-desoxy-mannonojirimycin mit 10 mmol 2,3- Dihydroindol umgesetzt.
$R_f$ = 0,7

Beispiel 59

3-(1,5-Didesoxy-1,5-imino-D-mannit-N-yl)-propionsäure-2,4-difluoranilid

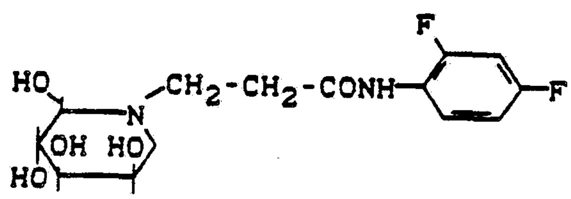

Entsprechend der Vorschrift aus Beispiel 1 werden 8,5 mmol N-(2-Carboxyethyl)-1-desoxy-mannonojirimycin mit 10 mmol 2,4- Difluoranilin umgesetzt.
$R_f$ = 0,65
$\alpha_D^{20}$ = -35° (C=1.0, Methanol)

Beispiel 60

3-(1,5-Didesoxy-1,5-imino-D-mannit-N-yl)-propionsäure-3,4-difluoranilid

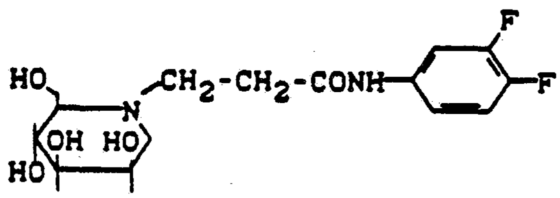

Entsprechend der Vorschrift aus Beispiel 1 werden 8,5 mmol N-(2-Carboxyethyl)-1-desoxy-mannonojirimycin mit 10 mmol 3,4- Difluoranilin umgesetzt.
$R_f$ = 0,64
$\alpha_D^{20}$ = -39° (C = 1.0, Methanol)

Beispiel 61

3-(1,5-Didesoxy-1,5-imino-D-mannit-N-yl)-propionsäure-2-trifluormethyl-anilid

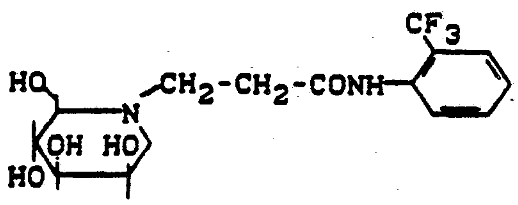

Entsprechend der Vorschrift aus Beispiel 1 werden 8,5 mmol N-(2-Carboxyethyl)-1-desoxy-mannonojirimycin mit 10 mmol 2- Trifluormethyl-anilin umgesetzt.
$R_f$ = 0,62

Beispiel 62

3-(1,5-Didesoxy-1,5-imino-D-mannit-N-yl)-propionsäure-2,6-difluoranilid

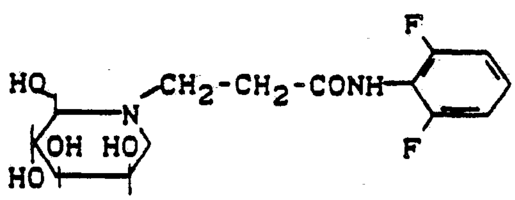

Entsprechend der Vorschrift aus Beispiel 1 werden 8,5 mmol N-(2-Carboxyethyl)-1-desoxy-mannonojirimycin mit 10 mmol 2,6- Difluoranilin umgesetzt.
$R_f$ = 0,62

Beispiel 63

3-(1,5-Didesoxy-1,5-imino-D-mannit-N-yl)-propionsäure-(adamantan-1-yl)-amid

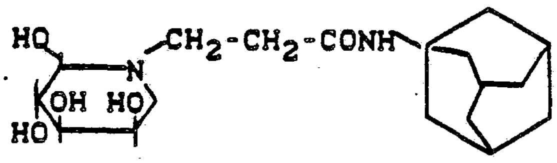

Entsprechend der Vorschrift aus Beispiel 1 werden 8,5 mmol N-(2-Carboxyethyl)-1-desoxy-mannonojirimycin mit 10 mmol 1-Amino- adamantan umgesetzt.
$R_f$ = 0,62

Beispiel 64

3-(1,5-Didesoxy-1,5-imino-D-mannit-N-yl)-propionsäure-3,4,5-trimethoxy-anilid

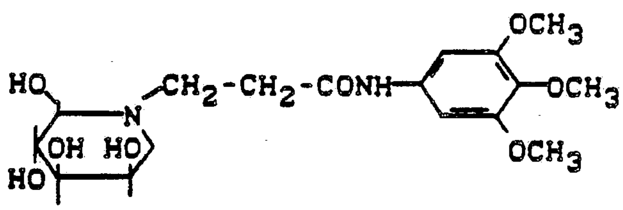

Entsprechend der Vorschrift aus Beispiel 1 werden 8,5 mmol N-(2-Carboxyethyl)-1-desoxy-mannonojirimycin mit 10 mmol 3,4,5- Trimethoxy-anilin umgesetzt.
$R_f$ = 0,68
$\alpha_D^{20}$ = -32° (C=1.0, Methanol)

Beispiel 65

3-(1,5-Didesoxy-1,5-imino-D-mannit-N-yl)-propionsäure-3,4-dimethoxy-anilid

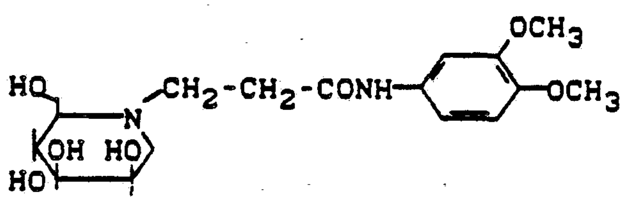

Entsprechend der Vorschrift aus Beispiel 1 werden 8,5 mmol N-(2-Carboxyethyl)-1-desoxy-mannonojirimycin mit 10 mmol 3,4- Dimethoxy-anilin umgesetzt.
$R_f$ = 0,65
$\alpha_D^{20}$ = -48° (C=1.0, Methanol)

Beispiel 66

3-(1,5-Didesoxy-1,5-imino-D-mannit-N-yl)-propionsäure-3,5-dimethoxy-anilid

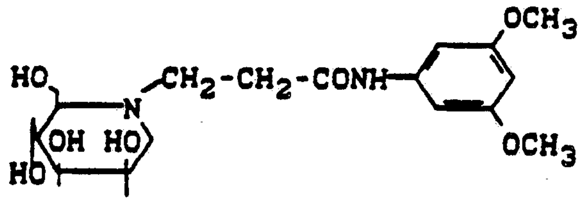

Entsprechend der Vorschrift aus Beispiel 1 werden 8,5 mmol N-(2-Carboxyethyl)-1-desoxy-mannonojirimycin mit 10 mmol 3,5- Dimethoxy-anilin umgesetzt.
$R_f$ = 0,68
$\alpha_D^{20}$ = -41° (C=1.0, Methanol)

Beispiel 67

3-(1,5-Didesoxy-1,5-imino-D-mannit-N-yl)-propionsäure-2,4,6-tribromanilid

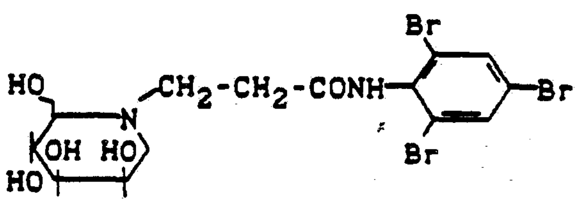

Entsprechend der Vorschrift aus Beispiel 1 werden 8,5 mmol N-(2-Carboxyethyl)-1-desoxy-mannonojirimycin mit 10 mmol 2,4,6- Tribromanilin umgesetzt.
$R_f$ = 0,62

Beispiel 68

3-(1,5-Didesoxy-1,5-imino-D-mannit-N-yl)-propionsäure-3-cyano-anilid

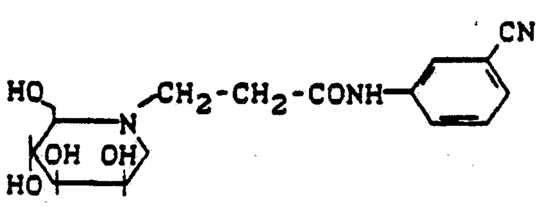

Entsprechend der Vorschrift aus Beispiel 1 werden 8,5 mmol N-(2-Carboxyethyl)-1-desoxy-mannonojirimycin mit 10 mmol 3-Cyano- anilin umgesetzt.
$R_f$ = 0,64
$\alpha_D^{20}$ = -49° (C=1.0, Methanol)

Beispiel 69

3-(1,5-Didesoxy-1,5-imino-D-mannit-N-yl)-propionsäure-4-cyclohexyl-anilid

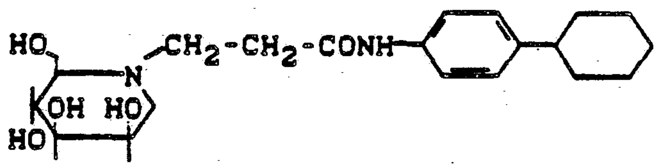

Entsprechend der Vorschrift aus Beispiel 1 werden 8,5 mmol N-(2-Carboxyethyl)-1-desoxy-mannonojirimycin mit 10 mmol 4-Cyclohexyl-anilin umgesetzt.
$R_f$ = 0,71
$\alpha_D^{20}$ = -38 (C = 1.0, Methanol)

Beispiel 70

3-(1,5-Didesoxy-1,5-imino-D-mannit-N-yl)-propionsäure-3,4-ethylendioxy-anilid

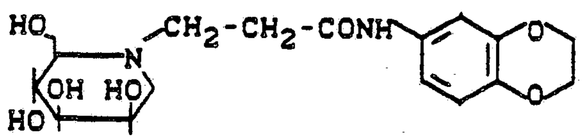

Entsprechend der Vorschrift aus Beispiel 1 werden 8,5 mmol N-(2-Carboxyethyl)-1-desoxy-mannonojirimycin mit 10 mmol 3,4- Ethylendioxy-anilin umgesetzt.
$R_f$ = 0,62
$\alpha_D^{20}$ = -43° (C = 1.0, Methanol)

Beispiel 71

3-(1,5-Didesoxy-1,5-imino-D-mannit-N-yl)-propionsäure-2-bromanilid

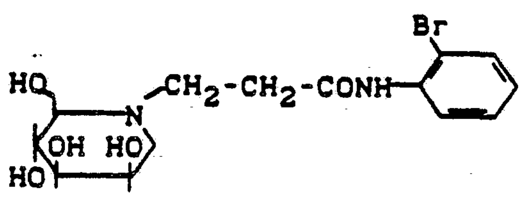

Entsprechend der Vorschrift aus Beispiel 1 werden 8,5 mmol N-(2-Carboxyethyl)-1-desoxy-mannonojirimycin mit 10 mmol 2- Bromanilin umgesetzt.
$R_f$ = 0,6

Beispiel 72

3-(1,5-Didesoxy-1,5-imino-D-mannit-N-yl)-propionsäure-3,4-methylendioxy-anilid

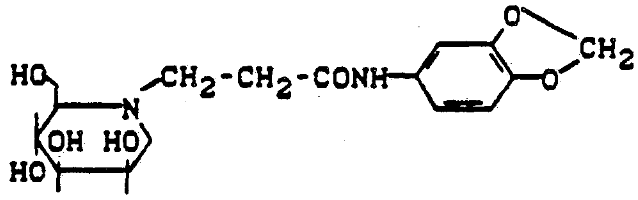

Entsprechend der Vorschrift aus Beispiel 1 werden 8,5 mmol N-(2-Carboxyethyl)-1-desoxy-mannonojirimycin mit 10 mmol 3,4- Methylendioxy-anilin umgesetzt.
$R_f$ = 0,62
$\alpha_D^{20}$ = -43° (C=1.0, Methanol)

Beispiel 73

3-(1,5-Didesoxy-1,5-imino-D-mannit-N-yl)-propionsäure-2-methylthio-anilid

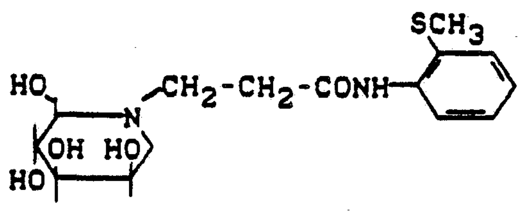

Entsprechend der Vorschrift aus Beispiel 1 werden 8,5 mmol N-(2-Carboxyethyl)-1-desoxy-mannonojirimycin mit 10 mmol 2- Methylthio-anilin umgesetzt.
$R_f$ = 0,68
$\alpha_D^{20}$ = -50° (C=1.0, Methanol)

Beispiel 74

3-(1,5-Didesoxy-1,5-imino-D-mannit-N-yl)-propionyl-4-(4-trifluormethyl-phenyl)-piperazin

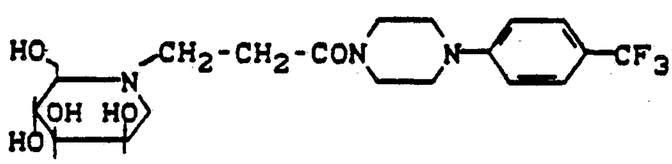

Entsprechend der Vorschrift aus Beispiel 1 werden 8,5 mmol N-(2-Carboxyethyl)-1-desoxy-mannonojirimycin mit 10 mmol 1-(4- Trifluormethylphenyl)-piperazin umgesetzt.
$R_f$ = 0,67

Beispiel 75

3-(1,5-Didesoxy-1,5-imino-D-mannit-N-yl)-propionsäure-anilid

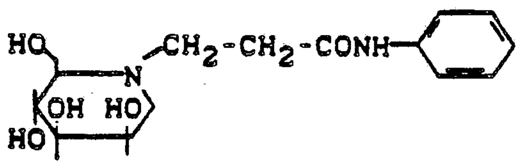

Entsprechend der Vorschrift aus Beispiel 1 werden 8,5 mmol N-(2-Carboxyethyl)-1-desoxy-mannonojirimycin mit 10 mmol Anilin umgesetzt.
$R_f$ = 0,52

Beispiel 76

3-(1,5-Didesoxy-1,5-imino-D-mannit-N-yl)-propionsäure-4-ethoxy-anilid

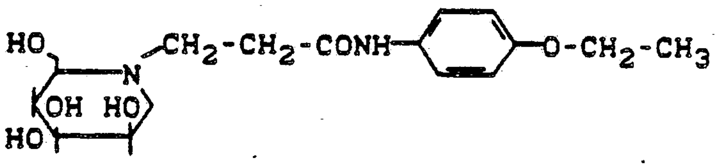

Entsprechend der Vorschrift aus Beispiel 1 werden 8,5 mmol N-(2-Carboxyethyl)-1-desoxy-mannonojirimycin mit 10 mmol 4-Ethoxy- anilin umgesetzt.
$R_f$ = 0,52
$\alpha_D^{20}$ = -46° (C=1.0, Methanol)

Beispiel 77

3-(1,5-Didesoxy-1,5-imino-D-mannit-N-yl)-propionsäure-2-dimethylaminoethyl-amid

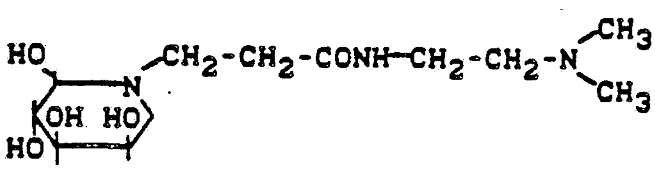

Entsprechend der Vorschrift aus Beispiel 1 werden 8,5 mmol N-(2-Carboxyethyl)-1-desoxy-mannonojirimycin mit 10 mmol 2- Dimethylaminoethylamin umgesetzt.
$R_f$ = 0,59

Beispiel 78

3-(1,5-Didesoxy-1,5-imino-D-mannit-N-yl)-propionsäure-[2-(3-methoxyphenyl)ethyl]amid

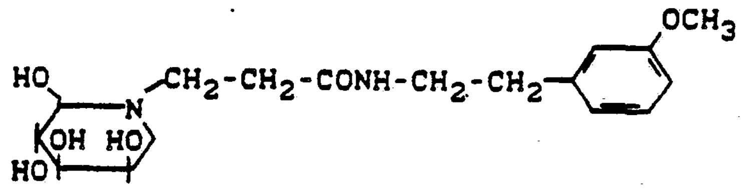

Entsprechend der Vorschrift aus Beispiel 1 werden 8,5 mmol N-(2-Carboxyethyl)-1-desoxy-mannonojirimycin mit 10 mmol 2-(3-Methoxyphenyl)ethylamin umgesetzt.
$R_f$ = 0,61

Beispiel 79

3-(1,5-Didesoxy-1,5-imino-D-mannit-N-yl)-propionsäure-2-pyridylmethyl-amid

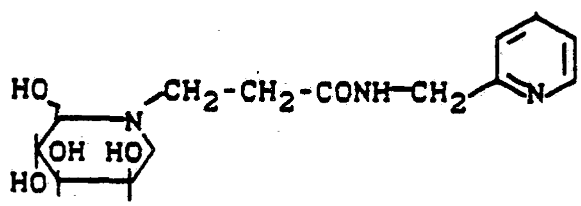

Entsprechend der Vorschrift aus Beispiel 1 werden 8,5 mmol N-(2-Carboxyethyl)-1-desoxy-mannonojirimycin mit 10 mmol 2- Aminomethyl-pyridin umgesetzt.
$R_f$ = 0,51
$\alpha_D^{20}$ = -15° (C=1.0, Methanol)

Beispiel 80

3-(1,5-Didesoxy-1,5-imino-D-mannit-N-yl)-propionyl-(4-cinnamyl)-piperazin

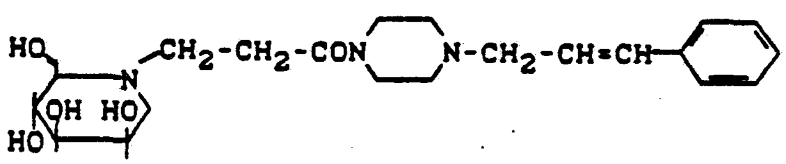

Entsprechend der Vorschrift aus Beispiel 1 werden 8,5 mmol N-(2-Carboxyethyl)-1-desoxy-mannonojirimycin mit 10 mmol 1- Cinnamyl-piperazin umgesetzt.
$R_f$ = 0,67

Beispiel 81

3-(1,5-Didesoxy-1,5-imino-D-mannit-N-yl)-propionsäure-4-(2-hydroxyethoxy)-anilid

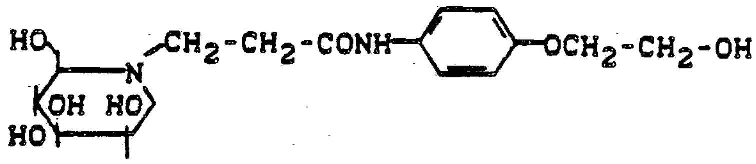

Entsprechend der Vorschrift aus Beispiel 1 werden 8,5 mmol N-(2-Carboxyethyl)-1-desoxy-mannonojirimycin mit 10 mmol 4-(2- Hydroxyethoxy)-anilin umgesetzt.
$R_f$ = 0,43

Beispiel 82

3-(1,5-Didesoxy-1,5-imino-D-mannit-N-yl)-propionyl-1-(4-p-fluorphenyl)-piperazin

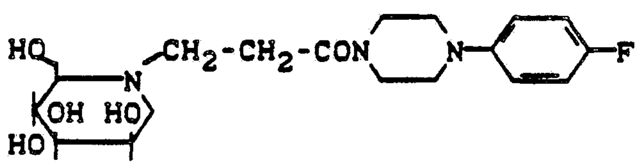

Entsprechend der Vorschrift aus Beispiel 1 werden 8,5 mmol N-(2-Carboxyethyl)-1-desoxy-mannonojirimycin mit 10 mmol 4-Fluorphenyl)-piperazin umgesetzt.
$R_f$ = 0,64
$\alpha_D^{20}$ = -23° (C=1.0, Methanol)

Beispiel 83

3-(1,5-Didesoxy-1,5-imino-D-mannit-N-yl)-propionitril

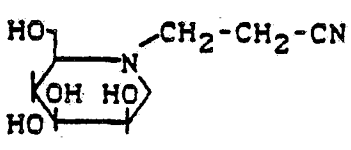

Man löst 326,4 g (2 mol) 1-Desoxy-mannonojirimycin in 5l einer 0,1 M wäßrigen von Kaliumhydrogenphosphat-Lösung und setzt soviel 1 N Natronlauge zu, bis sich pH 7,5 ergibt. Der Lösung weden 80 ml Acrylnitril zugesetzt. Diese Mischung wird 48 h bei 50° C gerührt. Nach dieser Zeit ist das Edukt nicht mehr nachzuweisen. (Dünnschichtchromatographie: $CH_2Cl_2$ : MeOH : $NH_3$-Lösung = 4:3:1, $R_f$ = 0,4). Zur Aufarbeitung werden Reagenz und Lösemittel im Hochvakuum verdampft. Der Rückstand wird in 5l einer Mischung aus Pyridin und Essigsäureanhydrid im Verhältnis 2:1 aufgenommen und 8 h gerührt. Nach dem Verdampfen des Lösemittels wird der Rückstand in 3 l Chloroform aufgenommen, die Lösung mehrfach mit Wasser extrahiert, über Natriumsulfat getrocknet und erneut eingeengt. Man erhält einen Sirup, der an 2 kg Kieselgel 60 im System $CH_2Cl_2$ : EtOH = 60 : 1 chromatographiert wird. Nach Verdampfung des Laufmittels wird der Rückstand in 3 l wasserfreiem Methanol, dem 0,1% Natriummethanolat zugesetzt werden, aufgenommen. Nach 8 h bei 40 ° C ist die Deblockierungsreaktion vollständig abgelaufen. Man neutralisiert durch Zugabe von 50 g eines stark sauren Ionenaustauschers, filtriert ab und engt langsam ein. Es kristallisieren 362 g (84%) Produkt.

$R_f$ = 0,4

$\alpha_D^{20}$ = -59 ° (C = 1.0, Methanol)

Beispiel 84

3-(1,5-Didesoxy-1,5-imino-D-mannit-N-yl)-propionsäure

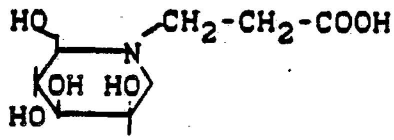

Man löst 50 g (0,23 Mol) der Verbindung aus Beispiel 83 in 1000 ml Wasser, setzt 8 g Bariumhydroxid zu und rührt 120 min. bei 80 ° C. Nach dem Erkalten wird durch Zugabe von 2 N Schwefelsäure neutral gestellt. Man trennt den Niederschlag durch Zentrifugieren ab und isoliert nach Verdampfung des Lösemittels 44,4 g (82%) Produkt.

$R_f$ = 0,1

$\alpha_D^{20}$ = -41 ° (C = 1, $H_2O$)

Beispiel 85

3-(1,5-Didesoxy-1,5-imino-D-glucit-N-yl)-propionyl-1-(4-phenyl)-piperazin

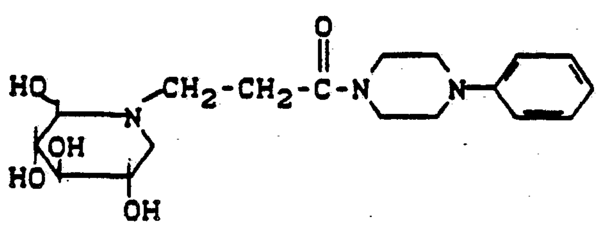

Entsprechend der Vorschrift aus Beispiel 1 werden 8,5 mmol N-(2-Carboxyethyl)-desoxynojirimycin mit 10 mmol 1-Phenyl-piperazin umgesetzt.

$R_f$ 0,65

Beispiel 86

3-(1,5-Didesoxy-1,5-imino-D-glucit-N-yl)-propionyl-1-(4-cyclopropyl)-piperazin

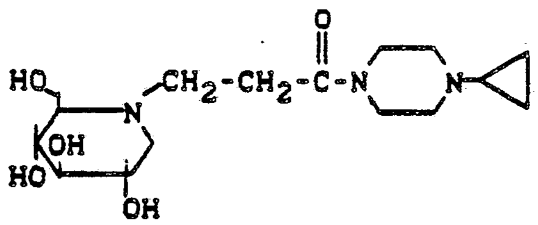

Entsprechend der Vorschrift aus Beispiel 1 werden 8,5 mmol N-(2-Carboxyethyl)-desoxynojirimycin mit 10 mmol 1-Cyclopropyl-piperazin umgesetzt.
$R_f$ 0,6

Beispiel 87

3-(1,5-Didesoxy-1,5-imino-D-glucit-N-yl)-propionyl-1-(4-tolyl)-piperazin

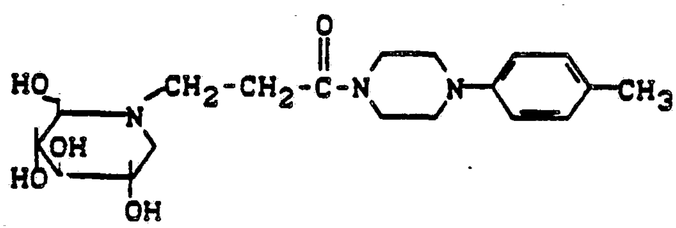

Entsprechend der Vorschrift aus Beispiel 1 werden 8,5 mmol N-(2-Carboxyethyl)-desoxynojirimycin mit 10 mmol 1-(4-Methylphenyl)-piperazin umgesetzt.
$R_f$ 0,66

Beispiel 88

3-(1,5-Didesoxy-1,5-imino-D-glucit-N-yl)-propionyl-1-(4-pyrrolidinoessigsäure-2-yl-)-piperazin

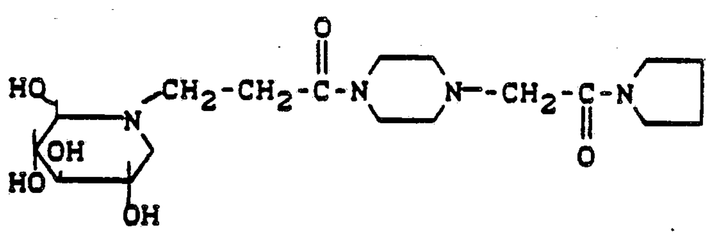

Entsprechend der Vorschrift aus Beispiel 1 werden 8,5 mmol N-(2-Carboxyethyl)-desoxynojirimycin mit 10 mmol Piperazino-essigsäurepyrrolidid umgesetzt.
$R_f$ 0,65

Beispiel 89

3-(1,5-Didesoxy-1,5-imino-D-glucit-N-yl)-propionsäure-(4-trifluormethyl-benzyl)-amid

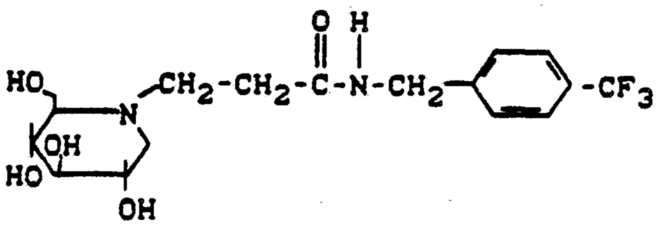

Entsprechend der Vorschrift aus Beispiel 1 werden 8,5 mmol N-(2-Carboxyethyl)-desoxynojirimycin mit 10 mmol 4-Trifluormethyl-benzylamin umgesetzt.
$R_f$ 0,65

Beispiel 90

3-(1,5-Didesoxy-1,5-imino-D-glucit-N-yl)-propionsäure-(2-phenyl-2-keto-ethyl)amid

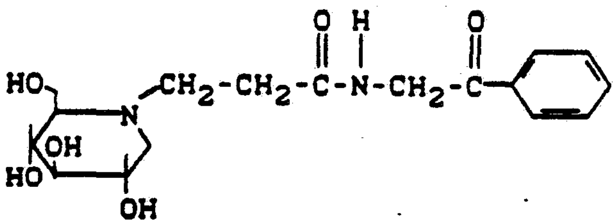

Entsprechend der Vorschrift aus Beispiel 1 werden 8,5 mmol N-(2-Carboxyethyl)-desoxynojirimycin mit 10 mmol ω-Amino-acetophenon umgesetzt.
$R_f$ 0,59

Beispiel 91

3-(1,5-Didesoxy-1,5-imino-D-glucit-N-yl)-propionsäure-(chinuclidin-3-yl)-amid

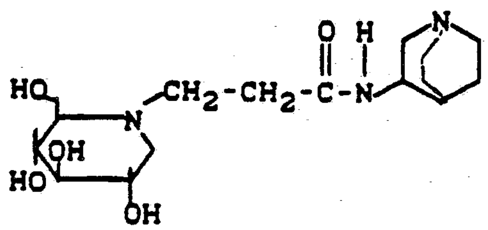

Entsprechend der Vorschrift aus Beispiel 1 werden 8,5 mmol N-(2-Carboxyethyl)-desoxynojirimycin mit 10 mmol 3-Amino-chinuclidin umgesetzt.
$R_f$ 0,65

Beispiel 92

3-(1,5-Didesoxy-1,5-imino-D-glucit-N-yl)-propionyl-1-(4-2-fluorphenyl)-piperazin

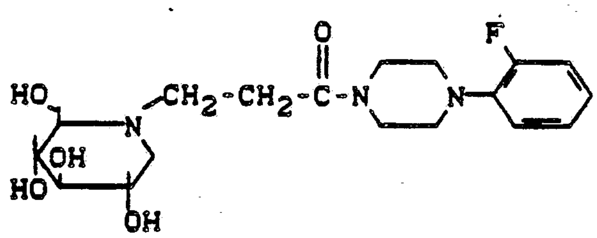

Entsprechend der Vorschrift aus Beispiel 1 werden 8,5 mmol N-(2-Carboxyethyl)-desoxynojirimycin mit 10 mmol 1-(2-Fluorphenyl)-piperazin umgesetzt.
$R_f$ 0,7

Beispiel 93

3-(1,5-Didesoxy-1,5-imino-D-glucit-N-yl)-propionyl-1-(3-methylphenyl)-piperazin

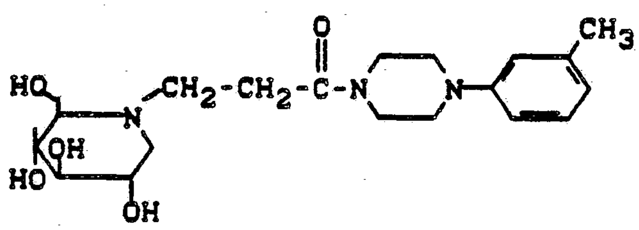

Entsprechend der Vorschrift aus Beispiel 1 werden 8,5 mmol N-(2-Carboxyethyl)-desoxynojirimycin mit 10 mmol 1-(3-Methylphenyl)-piperazin umgesetzt.
$R_f$ 0,65

Beispiel 94

3-(1,5-Didesoxy-1,5-imino-D-glucit-N-yl)-propionyl-1-(4-2-nitrophenyl)-piperazin

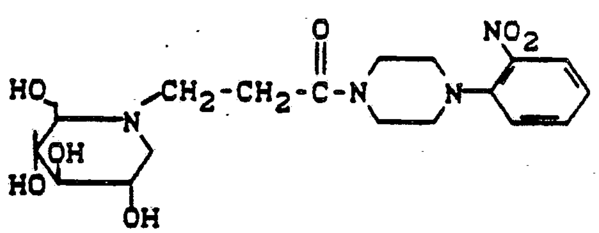

Entsprechend der Vorschrift aus Beispiel 1 werden 8,5 mmol N-(2-Carboxyethyl)-desoxynojirimycin mit 10 mmol 1-(2-Nitrophenyl)piperazin umgesetzt.

$R_f$ 0,65

Beispiel 95

3-(1,5-Didesoxy-1,5-imino-D-glucit-N-yl)-propionyl-1-(4-2-cyanophenyl)-piperazin

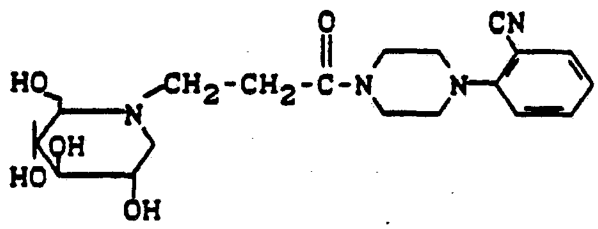

Entsprechend der Vorschrift aus Beispiel 1 werden 8,5 mmol N-(2-Carboxyethyl)-desoxynojirimycin mit 10 mmol 1-(2-cyanophenyl)-piperazin umgesetzt.
$R_f$ 0,65

Beispiel 96

3-(1,5-Didesoxy-1,5-imino-D-glucit-N-yl)-propionyl-1-(4-2-methylphenyl)-piperazin

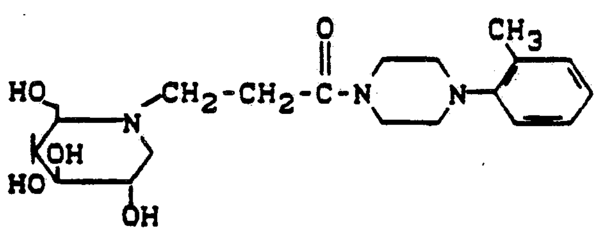

Entsprechend der Vorschrift aus Beispiel 1 werden 8,5 mmol N-(2-Carboxyethyl)-desoxynojirimycin mit 10 mmol 1-(2-Methylphenyl)-piperazin umgesetzt.
$R_f$ 0,6

Beispiel 97

3-(1,5-Didesoxy-1,5-imino-D-glucit-N-yl)-propionyl-1-(4-[3-trifluormethyl-4-chlor-phenyl])-piperazin

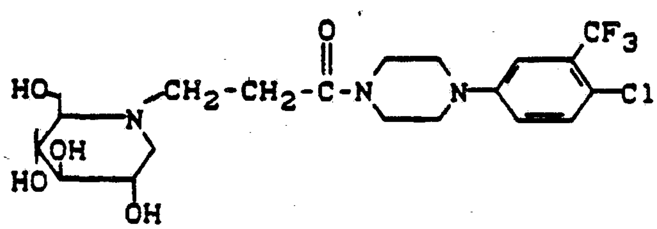

Entsprechend der Vorschrift aus Beispiel 1 werden 8,5 mmol N-(2-Carboxyethyl)-desoxynojirimycin mit 10 mmol 1-(3-Trifluormethyl-4-chlor-phenyl)-piperazin umgesetzt.
$R_f$ 0,65

Beispiel 98

3-(1,5-Didesoxy-1,5-imino-D-glucit-N-yl)-propionyl-1-(4-[morpholinoessigsäure-2-yl])-piperazin

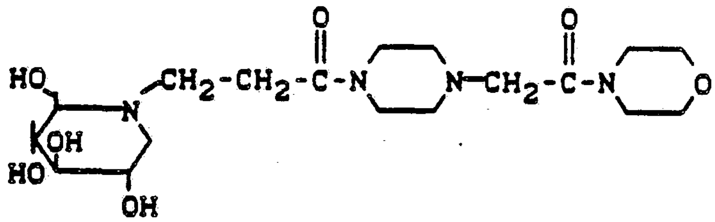

Entsprechend der Vorschrift aus Beispiel 1 werden 8,5 mmol N-(2-Carboxyethyl)-desoxynojirimycin mit 10 mmol Piperazino-essigsäuremorpholin umgesetzt.
$R_f$ 0,66

Beispiel 99

3-(1,5-Didesoxy-1,5-imino-D-glucit-N-yl)-propionyl-1-(4-[2-pyrimidyl])-piperazin

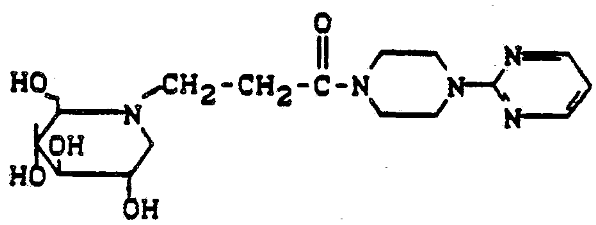

Entsprechend der Vorschrift aus Beispiel 1 werden 8,5 mmol N-(2-Carboxyethyl)-desoxynojirimycin mit 10 mmol 1-(2-Pyrimydyl)-piperazin umgesetzt.
$R_f$ 0,65

Beispiel 100

3-(1,5-Didesoxy-1,5-imino-D-glucit-N-yl)-propionyl-1-thiomorpholid

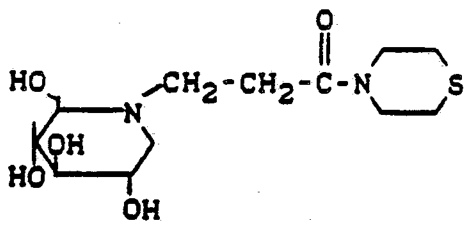

Entsprechend der Vorschrift aus Beispiel 1 werden 8,5 mmol N-(2-Carboxyethyl)-desoxynojirimycin mit 10 mmol Thiomorpholin umgesetzt.
$R_f$ 0,6

Beispiel 101

3-(1,5-Didesoxy-1,5-imino-D-glucit-N-yl)-propionsäure-(thiophen-2-yl)-methyl-amid

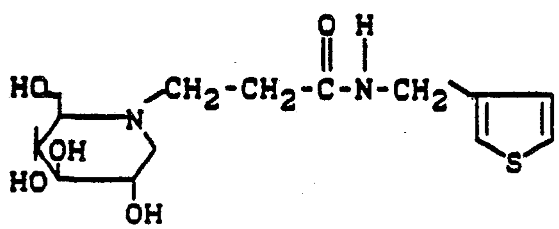

Entsprechend der Vorschrift aus Beispiel 1 werden 8,5 mmol N-(2-Carboxyethyl)-desoxynojirimycin mit 10 mmol 2-Aminomethyl-thiophen umgesetzt.
$R_f$ 0,59

Beispiel 102

3-(1,5-Didesoxy-1,5-imino-D-glucit-N-yl)-propionsäure-(1S)-1-phenylethylamid

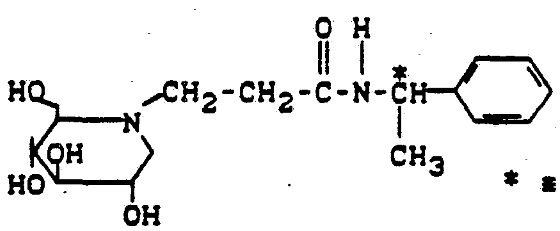

* ≡ S

Entsprechend der Vorschrift aus Beispiel 1 werden 8,5 mmol N-(2-Carboxyethyl)-desoxynojirimycin mit 10 mmol L(-)-1-Phenyl-ethylamin umgesetzt.
$R_f$ 0,65

Beispiel 103

3-(1,5-Didesoxy-1,5-imino-D-glucit-N-yl)-propionsäure-(1R)-phenylethylamid

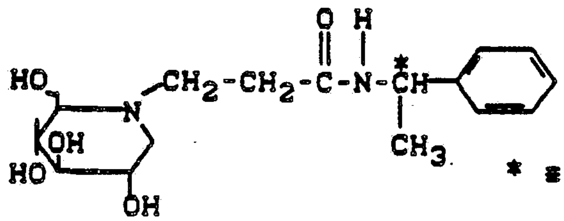

* ≡ R

Entsprechend der Vorschrift aus Beispiel 1 werden 8,5 mmol N-(2-Carboxyethyl)-desoxynojirimycin mit 10 mmol D(+)-1-Phenyl-ethylamin umgesetzt.
$R_f$ 0,65

## Beispiel 104

3-(1,5-Didesoxy-1,5-imino-D-glucit-N-yl)-propionsäure-butylamid

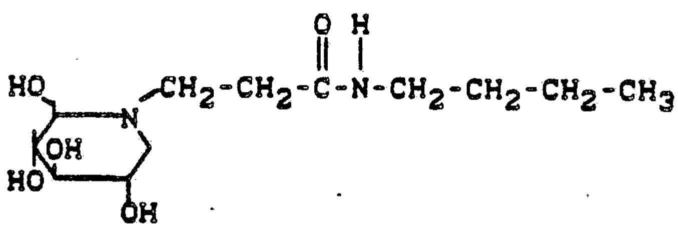

Entsprechend der Vorschrift aus Beispiel 1 werden 8,5 mmol N-(2-Carboxyethyl)-desoxynojirimycin mit 10 mmol Butylamin umgesetzt.
$R_f$ 0,62

## Beispiel 105

3-(1,5-Didesoxy-1,5-imino-D-glucit-N-yl)-propionsäure-t-butyl-amid

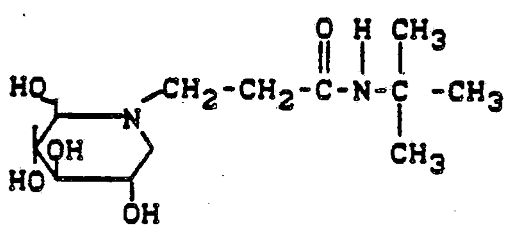

Entsprechend der Vorschrift aus Beispiel 1 werden 8,5 mmol N-(2-Carboxyethyl)-desoxynojirimycin mit 10 mmol t-Butylamin umgesetzt.
$R_f$ 0,6

Beispiel 106

3-(1,5-Didesoxy-1,5-imino-D-glucit-N-yl)-propionsäure-4-decyloxy-anilid

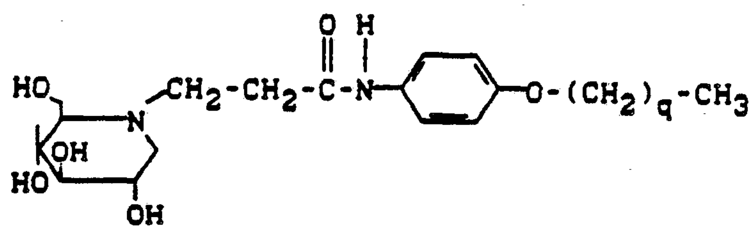

Entsprechend der Vorschrift aus Beispiel 1 werden 8,5 mmol N-(2-Carboxyethyl)-desoxynojirimycin mit 10 mmol 4-Decyloxy-anilin umgesetzt.
$R_f$ 0,8

Beispiel 107

4-(1,5-Didesoxy-1,5-imino-D-glucit-N-yl)-butansäure

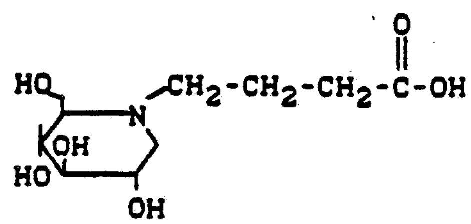

Man löst 1 Mol 1-Desoxynojirimycin in 1 l N,N-Dimethylformamid, setzt 0,5 Mol Kaliumcarbonat zu und tropft zu dieser Suspension unter Rühren 1,0 Mol ω-Brom-butansäureethylester. Sobald keine Gasentwicklung mehr zu beobachten ist, wird vom Festkörper abfiltriert und eingeengt. Der Rückstand wird in 1,05 l 1-N-Natriumhydroxidlösung aufgenommen und 8 h bei 90 °C gerührt. Sodann wird mit 1000 ml eines stark sauren Kationenaustauschers verrührt und abfiltriert.

Nach Evaporieren der Lösungsmittel isoliert man 0,75 Mol Produkt.
$R_f$ 0,25 $CH_2Cl_2$ = MeOH: $NH_3$-Lösung

Beispiel 108

4-(1,5-Didesoxy-1,5-imino-D-glucit-N-yl)-butansäure-4-ethoxy-anilid

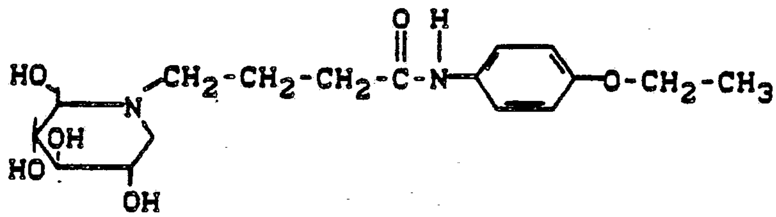

Entsprechend der Vorschrift aus Beispiel 1 werden 8,5 mmol N-(3-Carboxypropyl)-desoxynojirimycin mit 10 mmol 4-Ethoxy-anilin umgesetzt.
$R_f$ 0,68

Beispiel 109

4-(1,5-Didesoxy-1,5-imino-D-glucit-N-yl)-butansäure-4-t-butyl-anilid

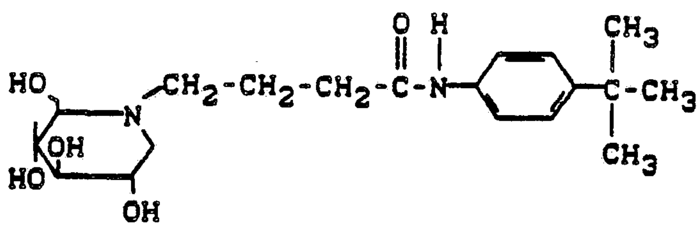

Entsprechend der Vorschrift aus Beispiel 1 werden 8,5 mmol N-(3-Carboxypropyl)-desoxynojirimycin mit 10 mmol 4-t-Butyl-anilin umgesetzt.
$R_f$ 0,6

Beispiel 110

4-(1,5-Didesoxy-1,5-imino-D-glucit-N-yl)-butansäure-4-(2-chlor-1,1,2-trifluorethoxy)-anilid

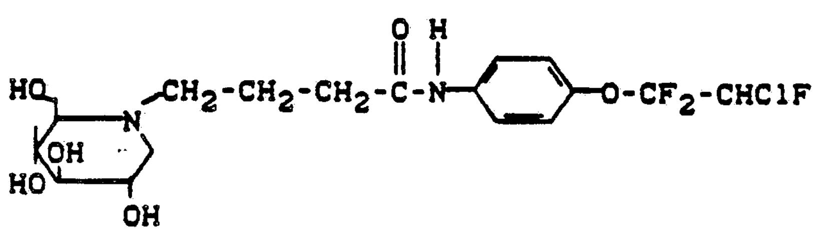

Entsprechend der Vorschrift aus Beispiel 1 werden 8,5 mmol N-(3-Carboxypropyl)-desoxynojirimycin mit 10 mmol 4-(2-Chlor-1,1,2-trifluorethoxy)-anilin umgesetzt.
$R_f$ 0,65

Beispiel 111

4-(1,5-Didesoxy-1,5-imino-D-glucit-N-yl)-butansäure-4-cyclohexyl-anilid

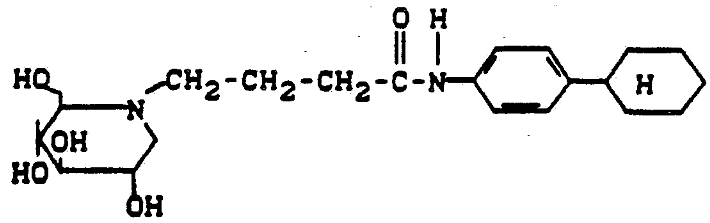

Entsprechend der Vorschrift aus Beispiel 1 werden 8,5 mmol N-(3-Carboxypropyl)-desoxynojirimycin mit 10 mmol 4-Cyclohexyl-anilin umgesetzt.
$R_f$ 0,7

Beispiel 112

1-[3-(1,5-Didesoxy-1,5-imino-D-glucit-N-yl)-propionyl]-1,2,3,4-tetrhydroisochinolin

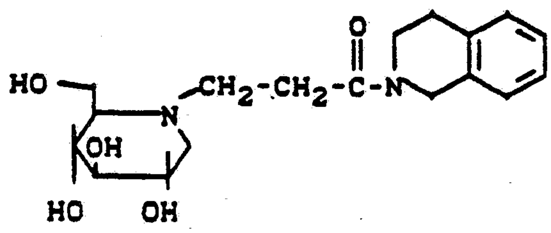

Entsprechend der Vorschrift aus Beispiel 1 werden 8,5 mmol N-(2-Carboxyethyl)-desoxynojirimycin mit 10 mmol = 1,2,3,4-Tetrahydroisochinolin umgesetzt.
$R_f$ 0,65

Beispiel 113

2- 1,5-Didesoxy-1,5-imino-D-glucit-N-yl ethansäuremethylester

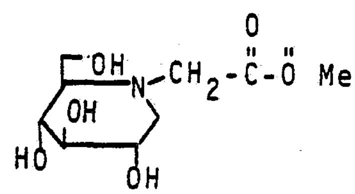

Einer Suspension von 163 g (1.0 Mol) 1-Desoxynijirimycin in 2000 ml trockenem N,N-Dimelthylformamid werden 69.1 g (0,5 Mol) Kaliumcarbonat zugesetzt. Die Suspension wird auf 0° abgekühlt. Sodann werden 153 g (1 Mol) 2-Brom-essigsäuremethylester über einen Zeitraum von 60 Min. zugesetzt.

Das Reaktionsgemenge wird bei 0° für weitere 10 Stunden gerührt. Zur Aufarbeitung wird von den Salzen abgesaugt und das Lösungsmittel verdampft. Das Rohprodukt wird unmittelbar dem nächsten Reaktionsschritt unterworfen.

$R_f$ 0.6 $CH_2Cl_2$:MeOH $H_3N$ aq = 4:3:1 V,V,V

Beispiel 114

2-(1,5-Didesoxy-1,5-imino-D-glucit-N-yl)ethansäure-anilid

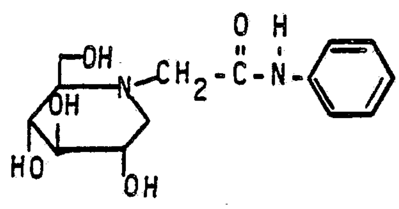

23.5 g (0.1 Mol) des Produktes aus Beispiel 113 und 9.3 g (0.1 Mol) frisch destilliertes Anilin werden 5 Stungden unter Inertgasatmosphäre auf 180° erhitzt.

Nach dieser Zeit wird das Reaktionsprodukt an Kieselgel adsorbiert und durch Chromatographie im System Toluol: Ethanol = 40:1 1:1 als mobile Phase eluiert.

$R_f$ 0.45

Beispiel 115:

2-(1,5-Didesoxy-1,5-imino-D-glucit-N-yl)-ethansäure-4-ethoxy-anilid

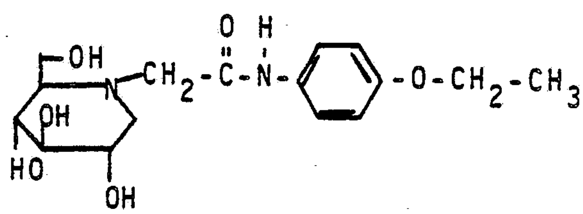

Entsprechend der Vorschrift aus Beispiel 114 werden 0.1 Mol 2-(1,5-Didesoxy-1,5-imino-D-glucit-N-yl) ethansäuremethylester mit 0.1 Mol 4-Ethoxyanilin verschmolzen und aufgearbeitet.

$R_f$ 0.45

Beispiel 116

2-(1,5-Didesoxy-1,5-imino-D-glucit-N-yl)-ethansäure-4-tert. butyl-anilid

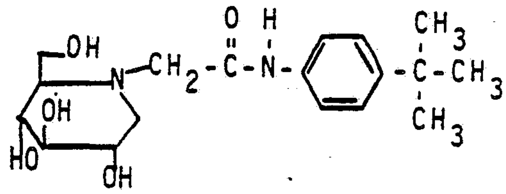

Entsprechend der Vorschrift aus Beispiel 114 werden 0.1 Mol 2-(1,5-Didesoxy-1,5-imino-D-glucit-N-yl)-ethansäuremethylester mit 0.1 Mol 4-tert. Butyl-anilin verschmolzen und aufgearbeitet.
$R_f$ 0.6

## Beispiel 117

2-(1,5-Didesoxy-1,5-imino-D-glucit-N-yl)-ethansäure-4-(2-hydroxyethoxy)-anilid

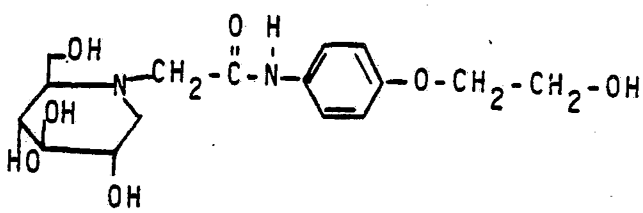

Entsprechend der Vorschrift aus Beispiel 114 werden 0.1 Mol 2-(1,5-Didesoxy-1,5-imino-D-glucit-N-yl)-ethansäuremethylester mit 0.1 Mol 4-(2-Hydroxyethoxy)anilin verschmolzen und aufgearbeitet.
$R_f$ 0.35

## Beispiel 118

2-(1,5-Didesoxy-1,5-imino-D-glucit-N-yl)ethansäure-4-hydroxy-anilid

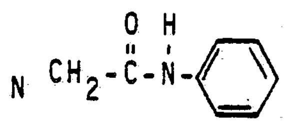

Entsprechend der Vorschrift aus Beispiel 114 wurden 0.1 Mol 2-(1,5-Didesoxy-1,5-imino-D-glucit-N-yl) ethansäuremethylester mit 0.1 Mol 4-Hydroxy-anilin verschmolzen und aufgearbeitet.
$R_f$ 0.55

**Ansprüche**

1. Verbindungen der allgemeinen Formel (I)

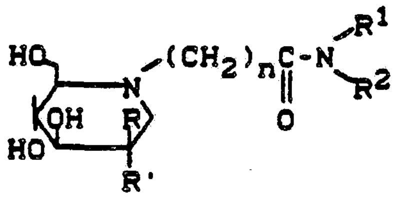

(I)

in welcher
R oder R′ für eine Hydroxylgruppe steht und jeweils andere R′ oder R für Wasserstoff steht,
n - für eine Zahl 1 bis 6 steht,
$R^1$ - für Wasserstoff steht, oder
- für geradkettiges oder verzweigtes Alkyl mit bis zu 3 Kohlenstoffatomen oder für Benzyl steht,
und
$R^2$ - für geradkettiges oder verzweigtes Alkyl mit bis zu 8 Kohlenstoffatomen steht, das substituiert sein kann durch Aryl mit 6 bis 10 Kohlenstoffatomen, wobei der Arylrest bis zu 4 gleiche oder verschiedene Substituenten der Reihe Fluor, Chlor, Brom, Iod, Cyano, Nitro, Alkyl, Alkoxy, Alkythio oder Arylthio mit jeweils jeweils bis zu 6 Kohlenstoffatomen, Dioxymethylen, Dioxyethylen, Trifluormethyl, Trifluormethoxy, Difluormethoxy, Carboxy oder Alkoxycarbonyl mit bis zu 4 Kohlenstoffatomen tragen kann, oder durch Pyridyl, Thienyl, Furyl, Pyrimidyl, Pyridazinyl, Pyrazinyl oder Chinolyl substituiert sein kann, oder
- für Cycloalkyl mit 3 bis 7 Kohlenstoffatomen steht, oder
- für Aryl mit 6 bis 10 Kohlenstoffatomen steht, wobei der Arylrest bis zu 4-fach gleich oder verschieden substituiert sein kann durch Fluor, Chlor, Brom, Iod, Cyano, Alkyl mit bis zu 6 Kohlenstoffatomen, Alkyloxy mit bis zu 12 Kohlenstoffatomen, Alkylsulfonyl mit jeweils bis zu 4 Kohlenstoffatomen, Trifluormethyl, Trifluormethoxy, Difluormethylen, Difluormethoxy, Trifluormethylthio, Dioxymethylen, Dioxyethylen, durch Cycloalkyl mit 3 bis 7 Kohlenstoffatomen, durch Carboxy oder Alkoxycarbonyl mit bis zu 4 Kohlenstoffatomen, durch Aryloxy mit 6 bis 10 Kohlenstoffatomen, wobei der Arylrest bis zu 4 gleiche oder verschiedene Substituenten der Reihe Fluor, Chlor, Brom, Iod, Cyano, Hydroxy, Nitro, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder durch Halogenalkoxy mit bis zu 6 Kohlenstoffatomen und bis zu 5 Fluor und/oder 3 Chloratomen, oder durch Hydroxyalkoxy, Hydroxyalkyl oder Cyanoalkyl mit jeweils bis zu 8 Kohlenstoffatomen, oder durch Alkenyloxy mit bis zu 6 Kohlenstoffatomen, oder durch eine Gruppe $-XR^3$,
wobei
X - eine geradkettige oder verzweigte Alkylen-oder Alkenylenkette mit bis zu 8 Kohlenstoffatomen bedeutet,
und
$R^3$ - Aryl mit 6 bis 10 Kohlenstoffatomen bedeutet, wobei der Arylrest bis zu 4 gleiche oder verschiedene Substitueten der Reihe Fluor, Chlor, Brom, Iod, Cyano, Alkyl, Alkoxy, Alkylthio mit jeweils bis zu 6 Kohlenstoffatomen, Nitro, Dioxymethylen, Dioxyethylen, Trifluormethyl, Trifluormethoxy, Difluormethoxy oder Trifluormethylthio tragen kann, oder
- einen 5- bis 6-gliedrigen gesättigten oder ungesättigten heterocyclischen Ring bedeutet, der benzokondensiert sein kann und der als Heteroatome Sauerstoff, Schwefel oder bis zu 2 Stickstoffatome enthalten kann, oder
- Hydroxy, Carboxy, Alkoxycarbonyl mit bis zu 6 Kohlenstoffatomen, Alkylcarbonyloxy mit bis zu 18 Kohlenstoffatomen, Cycloalkyl mit 3 bis 6 Kohlenstoffatomen bedeutet, oder
- eine Gruppe $-OSO_3H$, $-CONH-NH_2$, $-CONH_2$ oder Amino, Alkylamino oder Dialkylamino mit jeweils bis zu 6 Kohlenstoffatomen je Alkylgruppe bedeutet, oder
- Alkyl oder Hydroxyalkyl mit bis zu 8 Kohlenstoffatomen bedeutet,
oder
- für einen gesättigten, verbrückten Heterocyclus
oder
$R^1$ und $R^2$ gemeinsam mit dem Stickstoffatomen einen 5 bis 6-gliedrigen Ring bilden, der anelliert sein kann, der durch Sauerstoff, Schwefel oder die Gruppe $-NR^4$ unterbrochen sein kann, und der substituiert sein kann durch Alkyl mit bis zu 4 Kohlenstoffatomen, Hydroxy, Phenyl, Carboxy oder Alkoxycarbonyl mit bis zu 4 Kohlenstoffatomen,

wobei

$R^4$ - Aryl mit 6 bis 10 Kohlenstoffatomen bedeutet, wobei der Arylrest bis 4 gleiche oder verschiedene Substituenten der Gruppe Fluor, Chlor, Brom, Iod, Cyano, Nitro, Hydroxy, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder

- Alkoxycarbonyl mit bis zu 6 Kohlenstoff atomen bedeutet, oder
- Pyridyl, Pyrimidyl, Furyl, Thienyl, Pyrazinyl, Pyridazinyl oder Chinolyl bedeutet, oder
- Cycloalkyl mit 3 bis 7 Kohlenstoffatomen bedeutet, oder
- Alkyl oder Alkenyl mit bis zu 6 Kohlenstoffatomen bedeutet, das bis zu 2-fach gleich oder verschieden substituiert sein kann durch gegebenenfalls durch Chlor, Dioxymethylen oder Trifluormethyl substituiertes Phenyl, Hydroxy, Amino, Alkylamino, Dialkylamino mit jeweils bis zu 3 Kohlenstoffatomen je Alkylgruppe, Fluor, Chlor, Brom, Cycloalkyl mit 3 bis 6 Kohlenstoffatomen, durch Carboxy oder Alkoxycarbonyl mit bis zu 6 Kohlenstoffatomen, durch Pyridyl, Pyrimidyl, Pyrazinyl, Morpholinyl, Thiomorpholinyl, Pyrrolidinyl oder Pyrrolindocarbonyl, durch Morpholinocarbonyl, durch Alkylaminocarbonyl, Dialkylaminocarbonyl oder Phenylalkylaminocarbonyl mit jeweils bis zu 6 Kohlenstoffatomen je Alkylgruppe substituiert sein kann.

2. Verbindungen der allgemeinen Formel (I) gemäß Anspruch 1,

in welcher

n - für eine Zahl 1 bis 4 steht,

$R^1$ - für Wasserstoff steht,

und

$R^2$ - für geradkettiges oder verzweigtes Alkyl mit bis zu 6 Kohlenstoffatomen steht, das substituiert sein kann durch Pyridyl, Thienyl oder durch Phenyl, wobei der Phenylrest bis zu 3 gleiche oder verschiedene Substituenten der Reihe Fluor, Chlor, Brom, Hydroxy, Alkyl, Alkoxy mit bis zu 9 Kohlenstoffatomen, Dioxymethylen, Dioxyethylen oder Carboxy, oder

- für Cyclopentyl, Cyclohexyl oder Cycloheptyl steht, oder
- für Phenyl steht, das bis zu 3-fach gleich oder verschieden substituiert sein kann durch Fluor, Chlor, Brom, Cyano, Alkyl mit bis zu 4 Kohlenstoffatomen, Hydroxyalkyl oder Hydroxyalkoxy mit bis zu 4 Kohlenstoffatomen, Trifluormethyl, Trifluormethoxy, Dioxymethylen, Dioxyethylen, durch gegebenenfalls im Aromaten durch Fluor, Chlor, Brom, Hydroxy, Alkyl, Alkoxy, mit bis zu 9 Kohlenstoffatomen, Dioxymethylen, Trifluormethyl, oder Carboxy substituiertes Phenoxy, oder durch geradkettiges oder verzweigtes Alkyloxy mit bis zu 4 Kohlenstoffatomen, oder durch eine Gruppe $-XR^3$

wobei

X - eine geradkettige oder verzweigte Alkylenkette mit bis zu 6 Kohlenstoffatomen bedeutet,

und

$R^3$ - ein bis zu 3-fach gleich oder verschieden durch Fluor, Chlor, Brom, Hydroxy, Alkyl, Alkoxy, mit bis zu 4 Kohlenstoffatomen, Dioxymethylen, Trifluormethyl oder Carboxy substituiertem Phenyl bedeutet, oder

- Pyridyl, Morpholinyl oder Piperidinyl, bedeutet, oder
- Hydroxy, Carboxy, Alkoxycarbonyl mit bis zu 4 Kohlenstoffatomen, oder Cyclopropyl, Cyclopentyl oder Cyclohexyl bedeutet, oder
- Amino, Alkylamino oder Dialkylamino mit jeweils bis zu 4 Kohlenstoffatomen je Alkylgruppe bedeutet, oder

$R^1$ und $R^2$ gemeinsam mit dem Stickstoff einen Ring der Formel

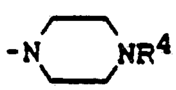 , 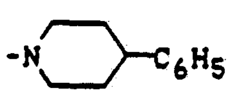 , 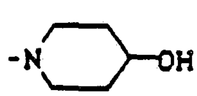 , 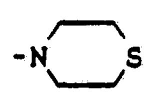

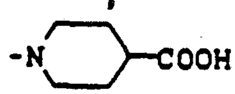 oder 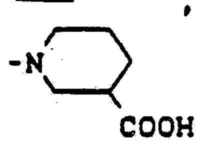 bilden,

wobei

$R^4$ - einen gegebenenfalls bis zu 3-fach gleich oder verschiedene durch Fluor, Chlor, Brom, Hydroxy, Alkyl, Alkoxy mit bis zu 4 Kohlenstoffatomen, Dioxymethylen, Trifluormethyl, oder Carboxy substituertes Phenyl bedeutet, oder

- Alkoxycarbonyl mit bis zu 4 Kohlenstoffatomen, bedeutet, oder

- Pyridyl oder Pyrimidyl bedeutet, oder
-Cyclopropyl, Cyclopentyl oder Cyclohexyl bedeutet, oder
- geradkettiges oder verzweigtes Alkyl oder Alkenyl mit bis zu 4 Kohlenstoffatomen bedeutet, das bis zu 2-fach gleich oder verschieden durch gegebenenfalls durch Chlor, Trifluormethyl oder Dioxymethylen substituiertes Phenyl, Hydroxy, Amino, Methylamino, Dimethylamino, Fluor, Chlor, Cyclopropyl, Cyclopentyl, Cyclohexyl oder Alkoxycarbonyl mit bis zu 4 Kohlenstoffatomen, durch Morpholinyl, Pyrrolidinyl, Morpholinocarbonyl, Dialkylamino der Phenylalkylamino mit jeweils bis zu 4 Kohlenstoffatomen substituiert sein kann.

3. Verbindungen der allgemeinen Formel (I) gemäß Anspruch 1,
in welcher
n- - für die Zahl 2 steht,
$R^1$ - für Wasserstoff steht,
und
- für geradkettiges oder verzweigtes Alkyl mit bis zu 3 Kohlenstoffatomen, das sibstituiert sein kann durch Thienyl oder Phenyl
$R^2$ - für Phenyl steht, das biz zu 3-fach gleich oder verschieden substituiert sein kann durch Fluor, Chlor, Brom, Cyano Alkyl mit bis zu 4 Kohlenstoffatomen, Hydroxyalkyl, Alkoxy mit bis zu 9 Kohlenstoffatomen, Hydroxyalkoxy mit bis zu 4 Kohlenstoffatomen, Alkylthio mit bis zu 4 Kohlenstoffatomen, Trifluormethyl, Trifluormethoxy,
- für Chinulidinyl oder für 1,2,3,4-Tetrahydroisochinolin
oder
$R^1$ und $R^2$ gemeinsam mit dem Stickstoff einen Ring der Formel

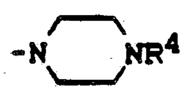 oder 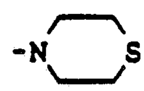 bilden ,

wobei
$R^4$ - einen gegebenenfalls bis zu 2-fach gleich oder verschiedene durch Fluor, Chlor, Brom, Hydroxy, Alkyl, Alkoxy mit bis zu 4 Kohlenstoffatomen, substituertes Phenyl bedeutet, oder - Alkoxycarbonyl mit bis zu s4 Kohlenstoffatomen bedeutet, oder
- Cyclopropyl, Cyclopentyl oder Cyclohexyl bedeutet, oder
- geradkettiges oder verzweigtes Alkyl oder Alkenyl mit bis zu 4 Kohlenstoffatomen bedeutet, das bis zu 2-fach gleich oder verschieden durch gegebenenfalls durch Hydroxy, Amino, Dimethylamino, Cyclopropyl oder Alkoxycarbonyl mit bis zu 4 Kohlenstoffatomen, durch Morpholinyl, Perrolidinyl oder Pyrrolidinocarbonyl substituiert sein kann.

4. Verbindungen aus der Gruppe
3-(1,5-Didesoxy-1,5-imino-D-glucit-N-yl)-propionsäure-(4-methoxy)-anilid,
3-(1,5-Didesoxy-1,5-imino-D-glucit-N-yl)-propionsäure-(4-methoxycarbonyl)-anilid,
3-(1,5-Didesoxy-1,5-imino-D-glucit-N-yl)-propionsäure-4-(4-pyridylmethoxy)-anilid,
3-(1,5-Didesoxy-1,5-imino-D-glucit-N-yl)-propionsäure-4-benzyloxy-anilid,
3-(1,5-Didesoxy-1,5-imino-D-glucit-N-yl)-propionsäure-4-trifluormethoxy-anilid,
3-(1,5-Didesoxy-1,5-imino-D-glucit-N-yl)-propionsäure-4-(2-oxocyclohexyloxy)-anilid,
3-(1,5-Didesoxy-1,5-imino-D-glucit-N-yl)-propionsäure-4-tert.butyl-anilid,
3-(1,5-Didesoxy-1,5-imino-D-glucit-N-yl)-propionsäure-4-(6-methyl-heptyloxy)-anilid,
3-(1,5-Didesoxy-1,5-imino-D-glucit-N-yl)-propionsäure-4-(2-N,N-diethyl-amino-ethoxy)-anilid,
3-(1,5-Didesoxy-1,5-imino-D-glucit-N-yl)-propionsäure-4-allyloxy-anilid,
3-(1,5-Didesoxy-1,5-imino-D-glucit-N-yl)-propionsäure-4-trifluormethyl-anilid,
3-(1,5-Didesoxy-1,5-imino-D-glucit-N-yl)-propionsäure-4-(2-chlor-1,1,2-trifluorethoxy)-anilid,
3-(1,5-Didesoxy-1,5-imino-D-glucit-N-yl)-propionsäure-(4-hydroxy)-anilid,
3-(1,5-Didesoxy-1,5-imino-D-glucit-N-yl)-propionsäure-4-isopropyl-anilid,
3-(1,5-Didesoxy-1,5-imino-D-glucit-N-yl)-propionsäure-2-isopropyl-anilid,
3-(1,5-Didesoxy-1,5-imino-D-glucit-N-yl)-propionsäure-4-fluor-anilid,
3-(1,5-Didesoxy-1,5-imino-D-glucit-N-yl)-propionsäure-2-fluor-anilid,
1-[3-(1,5-Didesoxy-1,5-imino-D-glucit-N-yl)-propionyl]-2,3-dihydroindol,
3-(1,5-Didesoxy-1,5-imino-D-glucit-N-yl)-propionsäure-2,4-difluoranilid,
3-(1,5-Didesoxy-1,5-imino-D-glucit-N-yl)-propionsäure-2,4-difluoranilid,

3-(1,5-Didesoxy-1,5-imino-D-glucit-N-yl)-propionsäure-2-trifluormethyl-anilid,
3-(1,5-Didesoxy-1,5-imino-D-glucit-N-yl)-propionsäure-2,6-difluoranilid,
3-(1,5-Didesoxy-1,5-imino-D-glucit-N-yl)-propionsäure-(adamantan-1-yl)-amid,
3-(1,5-Didesoxy-1,5-imino-D-glucit-N-yl)-propionsäure-3,4,5-trimethoxy-anilid,
3-(1,5-Didesoxy-1,5-imino-D-glucit-N-yl)-propionsäure-3,4-dimethoxy-anilid,
3-(1,5-Didesoxy-1,5-imino-D-glucit-N-yl)-propionsäure-3,5-dimethoxy-anilid,
3-(1,5-Didesoxy-1,5-imino-D-glucit-N-yl)-propionsäure-2,4,6-tribromanilid,
3-(1,5-Didesoxy-1,5-imino-D-glucit-N-yl)-propionsäure-3-cyano-anilid,
3-(1,5-Didesoxy-1,5-imino-D-glucit-N-yl)-propionsäure-4-cyclohexyl-anilid,
3-(1,5-Didesoxy-1,5-imino-D-glucit-N-yl)-propionsäure-3,4-ethylendioxy-anilid,
3-(1,5-Didesoxy-1,5-imino-D-glucit-N-yl)-propionsäure-2-bromanilid,
3-(1,5-Didesoxy-1,5-imino-D-glucit-N-yl)-propionsäure-3,4-methylendioxy-anilid,
3-(1,5-Didesoxy-1,5-imino-D-glucit-N-yl)-propionsäure-2-methylthio-anilid,
3-(1,5-Didesoxy-1,5-imino-D-glucit-N-yl)-propionsäure-2-methylthio-anilid,
3-(1,5-Didesoxy-1,5-imino-D-glucit-N-yl)-propionyl-4-(4-trifluormethyl-phenyl)-piperazin,
3-(1,5-Didesoxy-1,5-imino-D-glucit-N-yl)-propionsäure-anilid,
3-(1,5-Didesoxy-1,5-imino-D-glucit-N-yl)-propionsäure-4-ethoxy-anilid,
3-(1,5-Didesoxy-1,5-imino-D-glucit-N-yl)-propionsäure-2-dimethylaminoethyl-amid,
3-(1,5-Didesoxy-1,5-imino-D-glucit-N-yl)-propionsäure-[2-(3-methoxyphenyl)ethyl)-amid,
3-(1,5-Didesoxy-1,5-imino-D-glucit-N-yl)-propionsäure-2-pyridylmethyl-amid,
3-(1,5-Didesoxy-1,5-imino-D-glucit-N-yl)-propionyl-(4-cinnamyl)-piperazin,
3-(1,5-Didesoxy-1,5-imino-D-glucit-N-yl)-4-(2-hydroxyethoxy)-anilid,
3-(1,5-Didesoxy-1,5-imino-D-mannit-N-yl)-propionsäure-(4-methoxy)-anilid,
3-(1,5-Didesoxy-1,5-imino-D-mannit-N-yl)-propionsäure-(4-methoxycarbonyl)-anilid,
3-(1,5-Didesoxy-1,5-imino-D-mannit-N-yl)-propionsäure-4-(4-pyridylmethoxy)-anilid,
3-(1,5-Didesoxy-1,5-imino-D-mannit-N-yl)-propion-säure-4-benzyloxy-anilid,
3-(1,5-Didesoxy-1,5-imino-D-mannit-N-yl)-propionsäure-4-trifluormethoxy-anilid,
3-(1,5-Didesoxy-1,5-imino-D-mannit-N-yl)-propionsäure-4-(2-oxocyclohexyloxy)-anilid,
3-(1,5-Didesoxy-1,5-imino-D-mannit-N-yl)-propionsäure-4-tert.butyl-anilid,
3-(1,5-Didesoxy-1,5-imino-D-mannit-N-yl)-propionsäure-4-(6-methyl-heptyloxy)-anilid,
3-(1,5-Didesoxy-1,5-imino-D-mannit-N-yl)-propionsäure-4-(2-N,N-diethyl-amino-ethoxy)-anilid,
3-(1,5-Didesoxy-1,5-imino-D-mannit-N-yl)-propionsäure-4-allyloxy-anilid,
3-(1,5-Didesoxy-1,5-imino-D-mannit-N-yl)-propionsäure-4-trifluormethyl-anilid,
3-(1,5-Didesoxy-1,5-imino-D-mannit-N-yl)-propionsäure-4-(2-chlor-1,1,2-trifluorethoxy)-anilid,
3-(1,5-Didesoxy-1,5-imino-D-mannit-N-yl)-propionsäure-4-isopropyl-anilid,
3-(1,5-Didesoxy-1,5-imino-D-mannit-N-yl)-propionsäure-2-isopropyl-anilid,
3-(1,5-Didesoxy-1,5-imino-D-mannit-N-yl)-propionsäure-4-fluor-anilid,
3-(1,5-Didesoxy-1,5-imino-D-mannit-N-yl)-propionsäure-2-fluor-anilid,
1-[3-(1,5-Didesoxy-1,5-imino-D-mannit-N-yl)-propionyl]-2,3-dihydroindol,
3-(1,5-Didesoxy-1,5-imino-D-mannit-N-yl)-propionsäure-2,4-difluoranilid,
3-(1,5-Didesoxy-1,5-imino-D-mannit-N-yl)-propionsäure-2,4-difluoranilid,
3-(1,5-Didesoxy-1,5-imino-D-mannit-N-yl)-propionsäure-2-trifluormethyl-anilid,
3-(1,5-Didesoxy-1,5-imino-D-mannit-N-yl)-propionsäure-2,6-difluoranilid,
3-(1,5-Didesoxy-1,5-imino-D-mannit-N-yl)-propionsäure-(adamantan-1-yl)-amid,
3-(1,5-Didesoxy-1,5-imino-D-mannit-N-yl)-propionsäure-3,4,5-trimethoxy-anilid,
3-(1,5-Didesoxy-1,5-imino-D-mannit-N-yl)-propionsäure-3,4-dimethoxy-anilid,
3-(1,5-Didesoxy-1,5-imino-D-mannit-N-yl)-propionsäure-3,5-dimethoxy-anilid,
3-(1,5-Didesoxy-1,5-imino-D-mannit-N-yl)-propionsäure-3,5-dimethoxy-anilid,
3-(1,5-Didesoxy-1,5-imino-D-mannit-N-yl)-propionsäure-2,4,6-tribromanilid,
3-(1,5-Didesoxy-1,5-imino-D-mannit-N-yl)-propionsäure-3-cyano-anilid,
3-(1,5-Didesoxy-1,5-imino-D-mannit-N-yl)-propionsäure-4-cyclohexyl-anilid,
3-(1,5-Didesoxy-1,5-imino-D-mannit-N-yl)-propionsäure-3,4-ethylendioxy-anilid,
3-(1,5-Didesoxy-1,5-imino-D-mannit-N-yl)-propionsäure-2-bromanilid,
3-(1,5-Didesoxy-1,5-imino-D-mannit-N-yl)-propionsäure-3,4-methylendioxy-anilid,
3-(1,5-Didesoxy-1,5-imino-D-mannit-N-yl)-propionsäure-2-methylthio-anilid,
3-(1,5-Didesoxy-1,5-imino-D-mannit-N-yl)-propionsäure-2-methylthio-anilid,
3-(1,5-Didesoxy-1,5-imino-D-mannit-N-yl)-propionyl-4-(4-trifluormethyl-phenyl)-piperazin,
3-(1,5-Didesoxy-1,5-imino-D-mannit-N-yl)-propionsäure-anilid,

3-(1,5-Didesoxy-1,5-imino-D-mannit-N-yl)-propionsäure-4-ethoxy-anilid,
3-(1,5-Didesoxy-1,5-imino-D-mannit-N-yl)-propionsäure-2-dimethylaminoethyl-amid,
3-(1,5-Didesoxy-1,5-imino-D-mannit-N-yl)-propionsäure-[2-(3-methoxyphenyl)ethyl)-amid,
3-(1,5-Didesoxy-1,5-imino-D-mannit-N-yl)-propionsäure-2-pyridylmethyl-amid,
3-(1,5-Didesoxy-1,5-imino-D-mannit-N-yl)-propionyl-(4-cinnamyl)-piperazin und
3-(1,5-Didesoxy-1,5-imino-D-mannit-N-yl)-propionsäure-4-(2-hydroxyethoxy)-anilid,
3-(1,5-Didesoxy-1,5-imino-D-mannit-N-yl)-propionyl-1-(4-p-fluorphenyl)piperazin,
3-(1,5-Didesoxy-1,5-imino-D-mannit-N-yl)-propionylnitril
3-(1,5-Didesoxy-1,5-imino-D-mannit-N-yl)-propionsäure
1,5-Didexoxy-1,5-imino-D-mannit-N-yl)-propionyl-1-(4-phenyl)-piperazin
3-(1,5-Didesoxy-1,5-imino-D-mannit-N-yl)-propionyl-1(4-phenyl)-piperazin,
3-(1,5-Didesoxy-1,5-imino-D-glucit-N-yl)-propionyl-1-(4-cyclopropyl)-piperazin,
3-(1,5-Didesoxy-1,5-imino-D-glucit-N-yl)-propionsäure-(4-trifluormethyl-benzyl)-amid,
3-(1,5-Didesoxy-1,5-imino-D-glucit-N-yl)-propionsäure-(2-phenyl-2-Keto-ethyl)-amid,
3-(1,5-Didesoxy-1,5-imino-D-glucit-N-yl)-propionyl-1-(4-2-fluorphenyl)-piperazin,
3-(1,5-Didesoxy-1,5-imino-D-glucit-N-yl)-propionyl-1-(4-nitrophenyl)-piperazin,
3-(1,5-Didesoxy-1,5-imino-D-glucit-N-yl)-propionyl-1-(4-cyanophenyl)-piperazin,
3-(1,5-Didesoxy-1,5-imino-D-glucit-N-yl)-propionyl-1-(4-methylphenyl)-piperazin
3-(1,5-Didesoxy-1,5-imino-D-glucit-N-yl)-propionyl-1-(4-[3-trifluormethyl-4-chlorphenyl)-piperazin
3-(1,5-Didesoxy-1,5-imino-D-glucit-N-yl)propionyl-1-(4-[morpholinoessigsäure-2-yl])-piperazin,
3-(1,5-Didesoxy-1,5-imino-D-glucit-N-yl-propionyl-1-(4-[2-pyrimidyl])-piperazin,
3-(1,5-Didesoxy-1,5-imino-D-glucit-N-yl)-propionyl-1-thiomorpholid,
3-(1,5-Didesoxy-1,5-imino-D-glucit-N-yl)-propionsäure-(thiophen-2-yl)-methylamid,
3-(1,5-Didesoxy-1,5-imino-D-glucit-N-yl)-propionsäure-(1S)-1-phenylethylamid,
3-(1,5-Didesoxy-1,5-imino-D-glucit-N-yl) propionsäure-(1R)-phenylethylamid
3-(1,5-Didesoxy-1,5-imino-D-glucit-N-yl)-propionsäure-butylamid
3-(1,5-Didesoxy-1,5-imino-D-glucit-N-yl)-propionsäure-t-butylamid,
3-(1,5-Didesoxy-1,5-imino-D-glucit-N-yl)-propionsäure-4-decyloxyanilid,
4-(1,5-Didesoxy-1,5-imino-D-glucit-N-yl)-butansäure-4-ethoxy-anilid,
4-(1,5-Didesoxy-1,5-imino-D-glucit-N-yl)-butansäure-4-t-butyl-anilid
4-(1,5-Didesoxy-1,5-imino-D-glucit-N-yl)-butansäure-4-(2-chlor-1,1,2-trifluorethoxy)-anilid,
4-(1,5-Didesoxy-1,5-imino-D-glucit-N-yl)-butansäure-4-cyclohexyl-anilid,
1-[3-(1,5-Didesoxy-1,5-imino-D-glucit-N-yl)-propionyl]-1,2,3,4-tetrahydroisochinolin.

5. Verfahren zur Herstellung von Verbindungen der allgemeinen Formel (I)

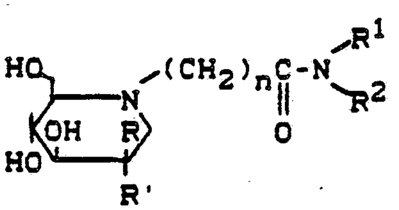

(I)

in welcher
R oder R′ für eine Hydroxylgruppe steht, und der jeweils andere R′ oder R für Wasserstoff steht,
n - für eine Zahl 1 bis 6 steht,
$R^1$ - für Wasserstoff steht, oder
- für geradkettiges oder verzweigtes Alkyl mit bis zu 3 Kohlenstoffatomen oder für Benzyl steht,
und
$R^2$ - für geradkettiges oder verzweigtes Alkyl mit bis zu 8 Kohlenstoffatomen steht, das substituiert sein kann durch Aryl mit 6 bis 10 Kohlenstoffatomen, wobei der Arylrest bis zu 4 gleiche oder verschiedene Substituenten der Reihe Fluor, Chlor, Brom, Iod, Cyano, Nitro, Alkyl, Alkoxy, Alkythio oder Arylthio mit jeweils bis zu 6 Kohlenstoffatomen, Dioxymethylen, Dioxyethylen, Trifluormethyl, Trifluormethoxy, Difluormethoxy, Carboxy oder Alkoxycarbonyl mit bis zu 4 Kohlenstoffatomen tragen kann, oder durch Pyridyl, Thienyl, Furyl, Pyrimidyl, Pyridazinyl, Pyrazinyl oder Chinolyl substituiert sein kann, oder
- für Cycloalkyl mit 3 bis 7 Kohlenstoffatomen steht, oder
- für Aryl mit 6 bis 10 Kohlenstoffatomen steht,

wobei der Arylrest bis zu 4-fach gleich oder verschieden substituiert sein kann durch Fluor, Chlor, Brom, Iod, Cyano, Alkyl mit bis zu 6 Kohlenstoffatomen, Alkyloxy mit bis zu 12 Kohlenstoffatomen, Alkylsulfonyl mit jeweils bis zu 4 Kohlenstoffatomen, Trifluormethyl, Trifluormethoxy, Difluormethylen, Difluormethoxy, Trifluormethylthio, Dioxymethylen, Dioxyethylen, durch Cycloalkyl mit 3 bis 7 Kohlenstoffatomen, durch Carboxy oder Alkoxycarbonyl mit bis zu 4 Kohlenstoffatomen, durch Aryloxy mit 6 bis 10 Kohlenstoffatomen, wobei der Arylrest bis zu 4 gleiche oder verschiedene Substituenten der Reihe Fluor, Chlor, Brom, Iod, Cyano, Hydroxy, Nitro, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder durch Halogenalkoxy mit bis zu 6 Kohlenstoffatomen und bis zu 5 Fluor und/oder 3 Chloratomen, oder durch Hydroxyalkoxy, Hydroxyalkyl oder Cyanoalkyl mit jeweils bis zu 8 Kohlenstoffatomen, oder durch Alkenyloxy mit bis zu 6 Kohlenstoffatomen, oder durch eine Gruppe $-XR^3$,

wobei

X - eine geradkettige oder verzweigte Alkylen-oder Alkenylenkette mit bis zu 8 Kohlenstoffatomen bedeutet, und

$R^3$ - Aryl mit 6 bis 10 Kohlenstoffatomen bedeutet, wobei der Arylrest bis zu 4 gleiche oder verschiedene Substituenten der Reihe Fluor, Chlor, Brom, Iod, Cyano, Alkyl, Alkoxy, Alkylthio mit jeweils bis zu 6 Kohlenstoffatomen, Nitro, Dioxymethylen, Dioxyethylen, Trifluormethyl, Trifluormethoxy, Difluormethoxy oder Trifluormethylthio tragen kann, oder

- einen 5- bis 6-gliedrigen gesättigten oder ungesättigten heterocyclischen Ring bedeutet, der benzokondensiert sein kann und der als Heteroatome Sauerstoff, Schwefel oder bis zu 2 Stickstoffatome enthalten kann, oder

- Hydroxy, Carboxy, Alkoxycarbonyl mit bis zu 6 Kohlenstoffatomen, Alkylcarbonyloxy mit bis zu 18 Kohlenstoffatomen, Cycloalkyl mit 3 bis 6 Kohlenstoffatomen bedeutet, oder

- eine Gruppe $-OSO_3H$, $-CONH-NH_2$, $-CONH_2$ oder Amino, Alkylamino oder Dialkylamino mit jeweils bis zu 6 Kohlenstoffatomen je Alkylgruppe bedeutet, oder

- Alkyl oder Hydroxyalkyl mit bis zu 8 Kohlenstoffatomen bedeutet,

oder

$R^1$ und $R^2$ gemeinsam mit dem Stickstoffatomen einen 5 bis 6-gliedrigen Ring bilden, der durch Sauerstoff oder die Gruppe $-NR^4$ unterbrochen sein kann, und der substituiert sein kann durch Alkyl mit bis zu 4 Kohlenstoffatomen, Hydroxy, Phenyl, Carboxy oder Alkoxycarbonyl mit bis zu 4 Kohlenstoffatomen,

wobei

$R^4$ - Aryl mit 6 bis 10 Kohlenstoffatomen bedeutet, wobei der Arylrest bis 4 gleiche oder verschiedene Substituenten der Gruppe Fluor, Chlor, Brom, Iod, Cyano, Nitro, Hydroxy, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder

- Alkoxycarbonyl mit bis zu 6 Kohlenstoffatomen bedeutet, oder
- Pyridyl, Pyrimidyl, Furyl, Thienyl, Pyrazinyl, Pyridazinyl oder Chinolyl bedeutet, oder
- Cycloalkyl mit 3 bis 7 Kohlenstoffatomen bedeutet, oder
- Alkyl oder Alkenyl mit bis zu 6 Kohlenstoffatomen bedeutet, das bis zu 2-fach gleich oder verschieden substituiert sein kann durch gegebenenfalls durch Chlor, Dioxymethylen oder Trifluormethyl substituiertes Phenyl, Hydroxy, Amino, Alkylamino, Dialkylamino mit jeweils bis zu 3 Kohlenstoffatomen je Alkylgruppe, Fluor, Chlor, Brom, Cycloalkyl mit 3 bis 6 Kohlenstoffatomen, durch Carboxy oder Alkoxycarbonyl mit bis zu 6 Kohlenstoffatomen, durch Pyridyl, Pyrimidyl, Pyrazinyl, Morpholinyl, Thiomorpholinyl, oder Pyrrolidinyl, durch Morpholinocarbonyl, durch Alkylaminocarbonyl, Dialkylaminocarbonyl oder Phenylalkylaminocarbonyl mit jeweils bis zu 6 Kohlenstoffatomen je Alkylgruppe substituiert sein kann,

dadurch gekennzeichnet, daß man Carbonsäuren der allgemeinen Formel (II)

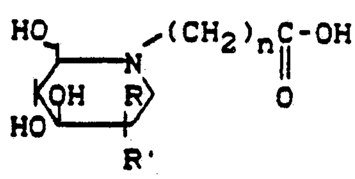 (II)

in welcher

n die oben angegebene Bedeutung hat,

nach bekannten Methoden, gegebenenfalls über ein reaktives Säurederivat, mit Aminen der allgemeinen

Formel (III)

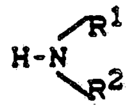 (III),

in welcher
$R^1$ und $R^2$ die oben angegebene Bedeutung haben,
gegebenenfalls in Anwesenheit eines inerten organischen Lösemittels umsetzt.

6. Verfahren nach Anspruch 5 worin die reaktiven Säurederivate ausgewählt sind aus der Gruppe enthaltend aktivierte Ester, Hydroxysuccinimidester, Säureimidazolide, Säurehalogenide, gemischte Anhydride oder Cyclohexylcarbodiimid.

7. Verwendung von Verbindungen gemäß den Ansprüchen 1 bis 4 zur Bekämpfung von Krankheiten.

8. Arzneimittel enthaltend eine oder mehrere der Verbindungen gemäß den Ansprüchen 1 bis 4.

9. Verwendung von Verbindungen gemäß den Ansprüchen 1 bis 4 für die Herstellung von Arzneimitteln zur Bekämpfung von Krankheiten.

10. Verfahren zur Herstellung von Arzneimitteln dadurch gekennzeichnet, daß man Verbindungen der Formel (I) gemäß den Ansprüchen 1 bis 4 gegebenenfalls unter Verwendung von üblichen Hilfs- und Trägerstoffen in eine geeignete Applikationsform überführt.

Europäisches Patentamt

# EUROPÄISCHER RECHERCHENBERICHT

Nummer der Anmeldung

EP 88 11 7369

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 193 770 (BAYER AG)<br>* Anspruch 1; Seiten 14,15 *<br>--- | 1 | C 07 D 211/46<br>C 07 D 401/12<br>C 07 D 401/06<br>C 07 D 405/12<br>C 07 D 453/02<br>C 07 D 409/12<br>A 61 K 31/445<br>A 61 K 31/505<br>A 61 K 31/47 |
| A,P | CHEMICAL ABSTRACTS, Band 107, Nr. 25, 21. Dezember 1987, Seite 27, Nr. 228527r, Columbus, Ohio, US; P.S. SUNKARA et al.: "Antiretroviral activity of castanospermine and deoxynojirimycin, specific inhibitors of glycoprotein processing", & BIOCHEM. BIOPHYS. RES. COMMUN. 1987, 148(1), 206-10<br>* Zusammenfassung *<br>--- | 7 | |
| A | CHEMICAL ABSTRACTS, Band 100, Nr. 1, 1984, Seite 293, Nr. 3381u, Columbus, Ohio, US; P.A. ROMERO et al.: "N-Methyl-1-deoxynojirimycin, a novel inhibitor of glycoprotein processing, and its effect on fowl plague virus maturation", & VIROLOGY 1983, 130(1), 238-42<br>* Zusammenfassung *<br>----- | 7 | **RECHERCHIERTE SACHGEBIETE (Int. Cl.4)**<br>C 07 D 211/00<br>C 07 D 401/00<br>C 07 D 405/00<br>C 07 D 453/00<br>C 07 D 409/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 24-01-1989 | CASADO Y MARTIN DE MERCA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)